(19) Europäisches Patentamt
European Patent Office
Office européen des brevets

(11) Veröffentlichungsnummer: **0 019 215**
**B1**

(12) # EUROPÄISCHE PATENTSCHRIFT

(45) Veröffentlichungstag der Patentschrift: **16.06.82**

(21) Anmeldenummer: **80102522.2**

(22) Anmeldetag: **08.05.80**

(51) Int. Cl.³: **C 02 F 11/00,** C 12 N 11/08, C 08 G 18/64, C 08 G 18/30, C 05 F 7/00, C 08 L 97/02

(54) **Verfahren zur Herstellung denaturierter Polyadditionsprodukte aus Isocyanat-Destillationsrückständen und Biomassen, sowie deren Verwendung als reaktiver Füllstoff, als Pflanzennährstoff und als Bindemittel zur Herstellung von Platten oder Formteilen.**

(30) Priorität: **21.05.79 DE 2920525**

(43) Veröffentlichungstag der Anmeldung: **26.11.80 Patentblatt 80/24**

(45) Bekanntmachung des Hinweises auf die Patenterteilung: **16.06.82 Patentblatt 82/24**

(84) Benannte Vertragsstaaten: **BE DE FR GB IT NL**

(56) Entgegenhaltungen:

**EP-A-0 010 243**
**DE-A-2 208 644**
**DE-A-2 410 693**
**FR-A-1 272 044**
**FR-A-2 314 196**
**US-A-3 976 465**
**US-A-4 021 368**

(73) Patentinhaber: **BAYER AG, Zentralbereich Patente, Marken und Lizenzen, D-5090 Leverkusen 1, Bayerwerk (DE)**

(72) Erfinder: **Reischl, Artur, Dr., H.T.-von-Böttinger-Strasse 19, D-5090 Leverkusen 1 (DE)**
Erfinder: **Wagner, Kuno, Dr., Am Kiesberg 8, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung denaturierter Polyadditionsprodukte aus Isocyanat-Destillationsrückständen und Biomassen, sowie deren Verwendung als reaktiver Füllstoff, als Pflanzennährstoff und als Bindemittel zur Herstellung von Platten oder Formteilen

Die Erfindung betrifft ein Verfahren zur irreversiblen Denaturierung von Mikroorganismen und deren Folgeprodukte enthaltenden Biomassen, insbesondere biologischen Klärschlämmen, unter gleichzeitiger Geruchsbefreiung durch Reaktion mit pulverisierten, bei der grosstechnischen Isocyanatherstellung anfallenden Destillationsrückständen. Die so erhaltenen, im wesentlichen geruchsfreien, denaturierten Polyadditionsprodukte werden erfindungsgemäss als reaktive Füllstoffe oder Formmassen bei der Herstellung von Kunststoffen, als Langzeitdünger in der Landwirtschaft oder als Bindemittel zur Herstellung von Platten und Formteilen beim Heissverpressen lignozellulosehaltiger Rohstoffe verwendet.

In biologischen Kläranlagen werden organisch-chemische Abwasserverunreinigungen mit Hilfe von Mikroorganismen abgebaut, d.h. biologisch eliminiert. Unter den dabei angewandten Bedingungen erfolgt eine besonders starke mikrobielle Vermehrung, wobei die Menge der hauptsächlich aus Bakterien bestehenden Biomasse in den sogenannten Belebtschlammbekken der Kläranlagen täglich um etwa 3 - 4 Gewichtsprozent zunimmt, so dass trotz Absterbens eines Teils der Mikroorganismen eine Verdoppelung der Bakterienmenge innerhalb 3 - 4 Wochen erfolgen würde. Ein Teil der Biomasse muss daher kontinuierlich den Belebtschlammbecken als sogenannter Überschussbelebtschlamm entnommen werden, um die optimalen Bedingungen der mikrobiellen Abwasserreinigung aufrechtzuerhalten. Deshalb fallen bei der vollbiologischen Reinigung industrieller und kommunaler Abwässer weltweit Biomassen in ausserordentlich grossen und stets steigenden Mengen an. Allein in der BRD werden zur Zeit jährlich bereits ca. 2 Millionen Tonnen (berechnet als Trockengewicht) solcher proteinhaltigen Biomassen entweder in Deponien abgeladen oder verbrannt. Die dafür erforderliche Entfernung des Wassers aus den Belebtschlämmen ist auch heute noch problematisch, da unter den in den Kläranlagen in der Praxis üblichen Sedimentationsbedingungen der abzuführende Belebtschlamm nur etwa 1 Gew.-% an mikrobieller Trockenmasse enthält. In üblichen Zentrifugen lässt sich der Feststoffgehalt des Schlamms nur auf 7 - 9 Gew.-% konzentrieren und auch durch Zugabe von Polyelektrolyten und Verwendung von Zentrifugaldekantern lediglich auf 12 - 15 Gew.-% steigern.

Die Belebtschlämme haben schon bei diesen niedrigen Konzentrationen wegen der starken chemischen und physikalischen Bindung des Wassers an die Mikroorganismen eine ausgeprägte Gelstruktur und verhältnismässig hohe Viskosität. Deshalb ist eine übliche Filtration ohne spezielle Behandlung unmöglich. Die Filtration wird auch dadurch erschwert, dass die Bakterienzellen einander anziehen und gemeinsame schleimige Hüllen ausbilden, wobei klebrige Flocken entstehen. Deshalb setzt man in der Praxis den Belebtüberschussschlämmen etwa die gleiche bis doppelte Menge an anorganischen Primärschlämmen zu, um die Entfernung des Wassers mit Hilfe von Filterpressen im technischen Massstab zu erleichtern. So wird ein Presskuchen mit einem sehr hohen Gehalt an anorganischen Bestandteilen und einem Wassergehalt von etwa 50 Gew.-%, bezogen auf die Gesamtmasse, gewonnen. Für die Verbrennung eignet sich andererseits nur Überschussbelebtschlamm-Pulver mit einem sehr hohen Gehalt an organischer Masse, entweder weitgehend wasserfrei mit dem Nachteil, dass der Trocknungsprozess weit mehr Energie erfordert, als an Wärmeäquivalent bei der Verbrennung gewonnen werden kann, oder aber man verbrennt wässrigen Belebtschlamm mit einem für die Verdampfung der mitgeführten Wassermengen entsprechenden Zusatz an beispielsweise Schweröl als Energieträger.

Ein weiteres Problem besteht darin, dass der Bakterien-Überschussschlamm, sobald er vom Klärbecken isoliert ist, sofort zu faulen beginnt und einen unerträglichen Geruch verbreitet. Sogar wasserfreies, bei 110°C getrocknetes Belebtschlamm-Pulver besitzt einen sehr unangenehmen Geruch und fault im feucht gewordenen Zustand weiter aus. Die Anwesenheit von pathogenen Keimen ist nicht auszuschliessen.

Die Kompostierung des Klärschlamms oder seine direkte Ausbringung als Düngemittel in der Landwirtschaft sind aus diesen Gründen nur in eng begrenztem Rahmen möglich. Schon jetzt ist also die Beseitigung bzw. Verwertung von Klärschlämmen ein grosses ökologisches Problem, welches trotz aller Anstrengungen bisher nicht zufriedenstellend gelöst werden konnte. Zum Stand der Technik gehörende Aufarbeitungsverfahren von Biomassen aus Mikroorganismen und Nachteile bzw. Unzulänglichkeiten dieser Verfahren werden in der älteren europäischen Patentanmeldung 10243 eingehend diskutiert.

Nach Berechnungen des Bundesministeriums des Inneren der BRD (Abfallwirtschaftsprogramm 1975 der Bundesregierung; Umweltbrief Nr. 13, 1976) wird die jährlich anfallende Klärschlamm-Menge bis 1985 jedoch auf ca. 50 Mio $m^3$ aus kommunalen und weiteren 30 Mio $m^3$ aus industriellen Anlagen steigen, was bei 95% Wassergehalt etwa 4 Millionen t Klärschlamm-Trockenmasse jährlich bedeutet. Sowohl aus ökologischen als auch aus ökonomischen Gründen ist es daher dringend erforderlich, verbesserte Verfahren zur Aufarbeitung von Belebtüberschussschlämmen unter Eliminierung von schädlichen Begleitstoffen aufzufinden und eine umweltfreundliche Verwertung der hauptsächlich aus hochwertigen Proteinen, Nukleinsäuren, Enzymen und anderen wertvollen organischen Verbindungen bestehenden Klärschlämme im Sinne eines im grosstechnischen Massstab anwendbaren Recycling-Prozesses zu ermöglichen.

Überraschenderweise wurde nunmehr gefunden, dass sich die verschiedensten Biomassen auf Basis von Mikroorganismen bzw. deren Stoffwechsel- und/oder Zersetzungsprodukten, insbesondere auch die oben beschriebenen Klärschlämme aus biologisch arbeitenden Kläranlagen, in einfacher und wesentlich verbesserter Weise aufarbeiten lassen, indem man sie mit Isocyanatgruppen aufweisenden Verbindungen, gegebenenfalls in Gegenwart von organischen Lösungsmitteln, Carbonylverbindungen, zur Aminoplast- bzw. Phenoplastbildung geeigneten Verbindungen und/oder weiteren Zusatzstoffen, gegebenenfalls bei erhöhter Temperatur und/oder erhöhtem Druck, zur Reaktion bringt. Unter «aufarbeiten» ist in diesem Zusammenhang insbesondere zu verstehen, dass die Biomassen aufkonzentriert, irreversibel denaturiert, geruchsfrei gemacht und auf diese Weise einer Verwertung in der kunststoffverarbeitenden Industrie und in der Landwirtschaft zugänglich gemacht werden. Die erfindungsgemäss erhaltenen Biomassen-Polyadditionsprodukte sind steril, in den meisten Fällen völlig geruchlos und enthalten die eingesetzte Biomasse chemisch gebunden und völlig denaturiert. Die Produkte verkleben in wässriger Phase nicht, sind ohne Komplikationen filtrierbar und lassen sich in energiesparender Weise trocknen. Sie sind völlig lagerstabil und frei von pathogenen Krankheitserregern. Bedingt durch totale Enzymdesaktivierung und vollständigen Zelltod der Biomassen werden Zersetzungs- bzw. Fäulnisvorgänge, Gärungen und unangenehme Geruchsbildung von enzymatisch oder mikrobiologisch abbaubaren Zellinhaltsstoffen vollständig unterbunden. Die Verfahrensprodukte sind daher bei beliebig langen Lagerzeiten in trockenem und auch in feuchtem Zustand bezüglich unangenehmer Geruchsbildung und weiterem enzymatischem Abbau vollständig stabilisiert.

Es sind bereits Verfahren bekanntgeworden, bei welchen Isocyanate mit anderen üblichen Ausgangsstoffen der Polyurethanchemie in Gegenwart biologisch aktiver Substanzen zu hochmolekularen Verbindungen umgesetzt werden. Im Gegensatz zur vorliegenden Erfindung, welche die Denaturierung von Biomassen auf Basis von Mikroorganismen unter völliger Zerstörung darin enthaltener lebender Zellen und aktiver Enzyme zum Ziel hat, wird in den bekannten Verfahren eine Fixierung ausgewählter biologisch wirksamer Verbindungen in Polyurethanen unter voller Erhaltung der Bioaktivität angestrebt. So wird z.B. in den DE-A-2 612 138 und DE-A-2 625 544 die Fixierung von Enzymen, Antigenen, Antikörpern oder Antibiotika mittels Isocyanatgruppen aufweisenden Vorpolymeren beschrieben. Die Polyadditionsreaktion muss dabei unter sehr schonenden Bedingungen ablaufen, damit eine Zerstörung der bioaktiven Substanzen vermieden wird. Die so erhaltenen Produkte finden als biospezifische Katalysatoren, Antigene oder Antikörper Verwendung. In analoger Weise können gemäss DE-A-2 319 706, DE-A-2 625 471, US-A-3 574 062, US-A-3 705 084, US-A-3 791 927, US-A-3 672 955 und US-A-3 929 575 verschiedenste an eine Polyurethanmatrix gebundene biologisch vollaktive oder sogar aktivierte Substanzen hergestellt werden.

Auch in der DE-A-2 310 693 wird ein Verfahren zum Immobilisieren von Enzymen beschrieben, indem man Mikroorganismuszellen mit einem Überschuss an Diisocyanaten umsetzt und an die noch freien NCO-Gruppen des Mikroorganimus/Diisocyanat-Adduktes Enzyme mittels deren reaktiven Gruppen immobilisierend anbindet. Die Enzyme bleiben dabei noch wirksam.

Wie bereits erwähnt, unterscheidet sich das erfindungsgemässe Verfahren hiervon nicht nur in den Ausgangsmaterialien (es werden keine isolierten biochemisch aktiven Einzelverbindungen sondern äusserst heterogen zusammengesetzte, im wesentlichen noch alle Zellbestandteile, im allgemeinen sogar eine im wesentlichen unversehrte Zellstruktur bzw. auch lebende Zellen enthaltende mikrobielle Biomassen eingesetzt) sondern auch in den Reaktionsbedingungen, so dass die erfindungsgemäss behandelten Biomassen in ihrer physikalischen, chemischen und biologischen Natur gegenüber dem Ausgangsmaterial völlig verändert sind.

In der US-A-4 021 368 werden «Biomassen», die als Bezeichnung für Mycele von Mikroorganismen (native oder getrocknete) verstanden werden und zur industriellen Retention von Schwermetallionen (U, Ra, Pb) eingesetzt werden sollen, dadurch zu einem versteiften, wasserunlöslichen Produkt umgewandelt, dass man die Mycele mit 10 bis 300 Gew.-% mit wenigstens einer polymerisierbaren Versteifungskomponente mischt, bei 90 - 105°C in den Polymerisations- und Trocknungsschritten behandelt und anschliessend granuliert. Die polymerisierbaren Versteifungskomponenten sind dabei besonders Formaldehyd, Resorcin, Harnstoff, m-Phenylendiamin, Gelatine, Stärke, Knochenleim, Casein, Epoxid-Harze und Acryl-Polymere. Neben Formaldehyd als Vernetzer werden in der Beschreibung auch Diisocyanate erwähnt.

In der DE-A-2 208 644 wird ein Verfahren zum Aufbereiten und Verfestigen von Sondermüll, einschliesslich Klärschlämmen, beschrieben, indem den Sondermüllarten organische Isocyanate, wie Toluylendiisocyanat, 4,4'-Diphenylmethandiisocyanat oder carbodiimidisierte Diphenylmethandiisocyanate zugemischt werden. Die anfallenden festen oder schaumstoffartigen Produkte lassen sich gut in Verbrennungsöfen beseitigen.

Es ist jedoch die Verwendung von Diisocyanaten zur Denaturierung von Biomassen, besonders von Klärschlämmen, nicht nur sehr unökonomisch, sondern es ist auch aus physiologischen (Dampfdruck der Diisocyanate) und Umweltschutzüberlegungen unerwünscht, solche niedermolekularen Diisocyanate zu verwenden.

Die erfindungsgemäss einzusetzenden Destillationsrückstände der Isocyanatherstellung sind praktisch frei von (Diisocyanat)-Monomeren. Sie sind auch ein Zwangsanfallsprodukt, das einer sinnvollen Verwendung zugeführt werden sollte. Es werden als Verfahrensprodukte weiterverwertbare Additionsprodukte erhalten, die sich für verschiedene Verwendungszwecke eignen.

Es war ferner überraschend, dass derart unlösliche, von monomeren Isocyanaten praktisch freie Rückstandsmassen eine solch wirksame Denaturierungsfunktion auszuüben vermögen.

Gegenstand der vorliegenden Erfindung ist somit ein Verfahren zur Herstellung von denaturierten Polyadditionsprodukten aus Biomassen und Isocyanaten sowie gegebenenfalls unter Zusatz weiterer polymerbildender Stoffe und nachträglicher Temperaturbehandlung, welches dadurch gekennzeichnet ist, dass man

A) 5 bis 98 Gew.-%, bevorzugt 20 bis 90 Gew.-%, bezogen auf A) + B), einer Biomasse auf Basis von Mikroorganismen bzw. deren Folge- und Zersetzungsprodukten mit

B) 95 bis 2 Gew.-%, bezogen auf A) + B) bei der grosstechnischen Isocyanat-Herstellung anfallende Destillationsrückstände, gegebenenfalls in Gegenwart von

C) Wasser und/oder einem organischen Lösungsmittel sowie gegebenenfalls in Gegenwart von

D) organischen und/oder anorganischen Zusatzstoffen, bei Temperaturen oberhalb von 50°C, vorzugsweise zwischen 50 und 200°C, besonders bevorzugt 80 bis 150°C, unter vollständiger Denaturierung umsetzt.

Gemäss einer besonderen Ausführungsform des erfindungsgemässen Verfahrens können mit den Biomassen vor oder nach oder simultan mit der Isocyanatpolyadditionsreaktion, gegebenenfalls unter partieller hydrolytischer Spaltung der Biomassen, Kondensationsreaktionen durchgeführt werden, indem man sie mit geeigneten Carbonylverbindungen, insbesondere Aldehyden oder N-Methylolgruppen enthaltenden Verbindungen, und gegebenenfalls weiteren zur Aminoplast- und/oder Phenoplastbildung befähigten Verbindungen umsetzt.

Unter «Biomassen» sind im Rahmen der vorliegenden Erfindung alle sich im Teilungszustand, Ruhezustand, partiellen oder vollständigen Zelltod, oder bereits in der enzymatischen Zersetzung oder in der Zersetzung durch Fremdkulturen befindlichen Biosysteme aus Mikroorganismen, wie Prokaryonten und Eukaryonten, z.B. Bakterien, Hefen, Protozoen und anderen Einzellern, Pilzen, Algen usw. zu verstehen. Beispiele hierfür sind

- Biomassen aus Mikroorganismen von biologisch arbeitenden Kläranlagen und andere Biomassen mikrobieller bzw. bakterieller Natur.
- Biomassen, wie sie anfallen

a) bei Verfahren zur Gewinnung von Produkten des primären Stoffwechsels, also z.B. bei der biotechnischen Herstellung von Äthanol, Butanol, Aceton, Zitronensäure, Milchsäure, Weinsäure, einfachen aliphatischen Carbonsäuren, Aminosäuren usw.,

b) bei technischen Fermentationsprozessen zur Herstellung von Produkten des sekundären Stoffwechsels, z.B. bei der Gewinnung von Antibiotika, Vitaminen, Wuchsstoffen, Steroidhormonen, Alkaloiden usw.,

c) bei Verfahren zur Gewinnung von Zellbestandteilen, wie Enzymen, Nucleinsäuren oder Polysacchariden, und

d) bei Verfahren zur Gewinnung von Hefen, z.B. zum Backen, zur alkoholischen Gärung oder zur Proteingewinnung aus Methan, Erdöl und Methanol.

- Biomassen, die bei Verfahren der Biotransformation anfallen, also bei Verfahren, bei denen Mikroorganismen als Katalysatoren organischchemischer Reaktionen, wie Oxidationen, Reduktionen, Decarboxylierungen, Phosphorylierungen, Aminierungen, Desaminierungen, Acetylierungen, Desacetylierungen usw. verwendet werden.

Für das erfindungsgemässe Verfahren bevorzugte Biomassen sind:

- Biomassen von biologisch arbeitenden Reinigungsanlagen von industriellen und kommunalen Abwässern. Derartige Biomassen bestehen aus zahlreichen Bakterien-, Algen- und Pilzarten, die optimal etwa bei einem P : N : C-Verhältnis von 1 : 5 : 100 arbeiten und als Omnivore (= Allesfresser) zu bezeichnen sind. Die aus Kläranlagen stammenden Biomassen, die auch als Klärschlämme od. Belebtschlämme bezeichnet werden, sind bei dem erfindungsgemässen Verfahren auch dann verwendbar, wenn sie Spuren an Quecksilber-, Cadmium-, Zink-, Eisen-, Chrom- und/oder Blei-Ionen enthalten.
- Faul- und Bioschlämme der verschiedensten Art sowie Biomassen mit hohen Anteilen an Escherichia coli und/oder verschiedensten pflanzlichen Schwebstoffen.
- Biomassen anaerober Faulung (Intensiv-Faulung), Müll-Klärschlamm-Kompostierungsprodukte, z.B. aus Verfahren der thermophilen Faulung (aerobthermophile Verfahren), ferner Produkte der aeroben Klärschlamm-Kompostierung nach dem System der Schnellrotte, weiterhin mikrobiell befallene Faserschlämme, Schlämme der Nahrungs- und Genussmittel-Industrie, z.B. Schlämme aus Molkereien und Schlachthöfen, sowie bereits getrocknete und in Deponien befindliche Bioschlämme.
- Die verschiedensten Hefearten (Pilzarten) aus technischen Prozessen, z.B. aus Fermentationsprozessen der alkoholischen Gärung.
- Biomassen der Essig-, Milchsäure-, Zitronensäure- oder Weinsäure-Herstellung, ferner Bakterienkulturen, die infolge enzymatischer Prozesse vergären.
- Fehlpartien von Hefekulturen.
- Biomassen der Proteinherstellung auf der Grundlage verschiedener Kohlenwasserstoff-Quellen, wie Erdöl, Paraffinverschnitten, Methan oder Methanol. Speziell in Frage kommen hierbei Biomassen auf Basis spezieller Hefezellen aus grosstechnischen Anlagen zur Erzeugung von Eiweiss aus Petroleumfraktionen und Fehlpartien derartiger Biomassen. Besonders geeignet sind in diesem Zusammenhang auch aus bakteriellen Mischkulturen bestehende Biomassen aus einzelligen Mikroorganismen, die bei der Eiweissgewinnung aus Erdgas (Methan) eingesetzt werden. Ferner sind geeignet Biomassen aus Pseudomonas-Bakterien, die im Fermenter bei etwa 37°C gezüchtet werden und aus Methanol als Kohlenstoffquelle proteinreiche Futtermittel erzeugen können.

- Biomassen der Penicillin-Herstellung, z.B. Penicillium notatum und Penicillium chrysogenum.
- Biomassen aus der Endstufe der Tetracyclin-Herstellung (Streptomyceten), Biomassen aus fadenartigen Bakterien der Sisomycin-Herstellung (Micromonospora), sowie andere Streptomyces-Arten.
- Biomassen auf Basis verschiedenster anderer Bakterien und Pilze, wie sie im Detail in der älteren europäischen Patentanmeldung 10243 beschrieben werden, und zahlreiche andere Biomassen mikrobieller Art wie sie in der Literatur beschrieben sind [vgl. Synthesis 4, 120 - 134 und 147 - 157 (1969)]. Diese Biomassen können aus Reinkulturen, aber selbstverständlich auch aus Mischkulturen, d.h. aus bei Fermentationsprozessen infizierten und daher unbrauchbaren Kulturen bestehen und z.B. auch in Mischung tote Zellen pflanzlicher Art oder Zellinhaltsstoffe wie Hemicellulosen enthalten.
- Algenarten, wie Blaualgen, Grünalgen (z.B. Chlorella), Kieselalgen, Jochalgen, Geisselalgen, Braunalgen und Rotalgen wie auch Protozoen.
- Mischkulturen der verschiedenartigsten Bakterien, Pilze und Algen, ebenso Kulturen von Biomassen, die mit andersartigen Pilzarten, Bakterienarten usw. infiziert sind und eine komplexe Zusammensetzung aufweisen. Beispielhaft genannt seien Mischkulturen, die sich an in Zersetzung befindlichen Trebern, Nährmedien wie Gelatine, Melassen, Stärken und anderen Polysacchariden an freier Luft und im feuchten Zustand sowie an proteinhaltigen, noch lebenden oder bereits in der Zersetzung befindlichen Algen ausbilden.

Im erfindungsgemässen Verfahren können auch Gemische verschiedener Biomassen eingesetzt werden. Ferner ist das erfindungsgemässe Verfahren auch denn anwendbar, wenn die Biomassen verschiedenartigste Verunreinigungen enthalten. Beispielsweise genannt seien in diesem Zusammenhang Biomassen, in denen Schwermetall-Salze, Pflanzenschutzmittel, Antibiotika oder andere organische oder anorganische Chemikalien vorhanden sind.

Besonders bevorzugt werden für die erfindungsgemässen Isocyanat-Polyadditionsreaktionen die schon eingehend beschriebenen wässrigen oder getrockneten pulverförmigen Klärschlämme aus industriellen und kommunalen Kläranlagen verwendet. Diese besitzen keine definierte Zusammensetzung, sondern bestehen je nach Abwasserverunreinigung und biologichen Bedingungen aus vielen Bakterienarten, Pilzen und Protozoen, von denen nur wenige beispielhaft genannt seien: Aerobacter aerogenes, Corynebacterium laevaniformas, Paracolobactrum aerogenoides, Escheria intermedium, Escheria faecale, Flavobakterien, Pseudomonas-, Nitrosomonas- und Nitrobacterarten, sowie Shaerotilus natens und weisse Schwefelbakterien. Darüber hinaus sind auch Enzyme, Fermente und Algen anwesend.

Die im erfindungsgemässen Verfahren einzusetzenden Biomassen enthalten die verschiedenartigsten Verbindungen mit H-aciden Gruppen, welche mit Isocyanaten Polyadditionsreaktionen eingehen können [siehe z.B. auch «Handbuch der Frischwasser- und Abwasserbiologie», Bd. II, S. 620 (1960) von H. Lubmann]. Beispiele hierfür sind u.a. alle Proteine, z.B. Lipoproteine, Glykoproteide als Bestandteile von Enzymen, die Enzyme selbst, wie Glukoseoxidase, Katalase, Glukose-Isomerase, Invertase, Lactase, Naringinase, Lipasen, Asparaginasen, α-Amylasen und Glykoamylasen, Cellulasen, Lysozyme, Proteasen usw.; Nucleoproteide; Ribonucleinsäuren und Desoxyribonucleinsäuren; Phosphatide, insbesondere Inositphosphatid, Colamin-kephalin und Serin-kephalin; Lipoide oder Plasmalogene, soweit sie als Base Colamin in Form eines Phosphorsäureesters gebunden enthalten; alle Zukker und polysaccharidartigen Zellreserve- und Zellinhaltsstoffe, Hemicellulosen, Stärken, Pektine und Lignine; Bestandteile der Zellwände von Bakterien, beispielsweise Polymere von Aminozuckern (Acetylglykosamin + N-Acetylmuraminsäure), die im N-Acetylmuraminsäure-Anteil über Polypeptide vernetzt sind; Zellwandbestandteile von Pilzen und Algen, wie z.B. Cellulosen, Hemicellulosen und andere Polysaccharide und Chitinanteile mit Acetyl-glukosamin- bzw. Acetylgalaktosamin-Anteilen.

Erfindungsgemäss ist es möglich, als Komponente B) Isocyanatgruppen aufweisende Produkte einzusetzen, welche unschmelzbar bzw. unlöslich sind.

Ökonomisch von besonderem Interesse ist es, für die erfindungsgemässe Denaturierung von Biomassen die bei der grosstechnischen Isocyanatherstellung anfallenden Destillationsrückstände zu benützen, für die bisher — ähnlich wie dies oben im Zusammenhang mit den Biomassen erläutert wurde — noch kein praktischer Anwendungszweck gefunden werden konnte und deren Beseitigung bisher ebenfalls erhebliche Probleme aufwarf (siehe in diesem Zusammenhang z.B. DE-A-2 846 815 und 2 846 809). Für die vorliegende Erfindung kommen dabei insbesondere die im wesentlichen monomerenfreien, vernetzten, in inerten organischen Lösungsmitteln unlöslichen, nicht unzersetzt schmelzbaren Destillationsrückstände in Betracht, wie sie bei der destillativen Entfernung von monomeren Toluylendiisocyanaten aus dem rohen Phosgenierungsprodukt von Toluylendiaminen, gegebenenfalls nach dem Einrühren in Wasser, als Schlakke anfallen und die vor ihrer Verwendung zu einem Pulver vermahlen und gegebenenfalls gleichzeitig und/oder anschliessend durch Reaktion mit gegenüber den funktionellen Gruppen des Destillationsrückstands, insbesondere den Isocyanatgruppen, reaktiven Verbindungen chemisch modifiziert wurden.

Diese im erfindungsgemässen Verfahren einzusetzenden Destillationsrückstände fallen, wie erwähnt, zwangsweise bei den heute gängigen Produktionsverfahren von 2,4- und/oder 2,6-Toluylendiisocyanat in grosstechnischem Massstab an. Es handelt sich dabei um höhermolekulare, über Hauptvalenzbindungen vernetzte Rückstandsschlacken, die meistens in einer Menge von über 10 Gew.-%, bezogen auf die berechnete quantitative Ausbeute an monomeren Diisocyanaten, entstehen und die zwecks besserer Handhabbarkeit meist über 150°C heiss in Wasser eingetragen werden, wobei unter Reaktion eines grossen Teils der freien Isocyanatgruppen zu Polyharnstoffgruppen eine grobteilige, unregelmässig geformte, unlösliche Schlacke entsteht. Diese Schlacke hat zwar noch einen Gehalt an freien NCO-Gruppen (im allgemeinen unter 15 Gew.-%, meist 1 bis 10 Gew.-%), jedoch ist sie praktisch frei an monomeren Diisocyanaten. Neben den NCO-Gruppen weisen die TDI-Rückstandsschlacken in unterschiedlichen Mengenverhältnissen Harnstoff- Biuret-, Uretdion-, Isocyanurat-, Carbodiimid-, Uretonimin- und gegebenenfalls auch Methylbenzimidazolongruppen sowie deren Biuretisierungsprodukte auf. Die Schlacken sind über diese verschiedenen funktionellen Gruppen so hoch vernetzt, dass sie selbst bei einer mittleren Teilchengrösse von weniger als 5 µm in inerten organischen Lösungsmitteln wie Methylenchlorid, Cyclohexan, Cyclohexanon, Toluol, Xylol oder Dichlorbenzol auch bei Siedetemperatur praktisch unlöslich sind. In siedendem Dimethylformamid werden die Rückstandspulver teilweise angequollen, lösen sich jedoch nicht. Beim Erhitzen erweicht, wenn überhaupt, nur ein sehr geringer Anteil der erfindungsgemäss einzusetzenden TDI- Destillationsrückstände oberhalb von ca. 250°C; jedoch tritt oberhalb von ca. 280°C Zersetzung unter Gasentwicklung ein, ohne dass die Destillationsrückstände schmelzen.

Einige der genannten Gruppierungen der TDI-Schlacke, beispielsweise Uretdion- und Carbodiimidgruppen, können zusätzlich bei erhöhter Temperatur mit den Biomassen chemisch reagieren.

Die, gegebenenfalls mit Wasser benetzte oder beim Denaturierungsprozess im Rührkessel in Wasser suspendierte, sehr grobkörnige TDI-Rückstandsschlacke wird zunächst vorzugsweise mit Hilfe einer Zerkleinerungsmaschine, beispielsweise mit einem Schneidgranulator oder einer Hammermühle, auf weniger als 3 mm vorzerkleinert und anschliessend zu einem beliebigen Zeitpunkt nach bekannten Mahlverfahren entweder in der Nass- oder Trockenphase auf eine für die vorgesehene Verwendung erforderliche Endfeinheit gebracht.

Wenn die TDI-Rückstände, beispielsweise beim grosstechnisch praktizierten, oben beschriebenen Denaturierungsprozess, in Wasser oder mit Wasser benetzt anfallen und vorgesehene Folgereaktionen mit in Wasser suspendierten Biomassen ausgeführt werden können, bietet sich besonders ökonomisch und umweltfreundlich eine Nasszerkleinerung der TDI-Grobschlakke in der wässrigen Biomassen-Suspension in diskontinuierlich oder kontinuierlich arbeitenden, gegebenenfalls zweistufig hintereinander geschalteten Maschinen an. Die Feststoffkonzentrationen dieser Mischungen liegen während der Nassmahlung vorzugsweise zwischen 10 und 45 Gew.-%. Neben Rohr- und Kugelmühlen kommen besonders vorteilhaft Zahnkolloid-, Trigonal-Zahnring-, Korundscheiben- und Rührwerkskugelmühlen in Frage, je nachdem, welche Korngrösse angestrebt wird.

In speziellen Fällen kann beim Mahlvorgang ein Teil oder das gesamte Wasser durch eine andere Flüssigkeit ersetzt werden, die jeweils nach dem späteren Verwendungzweck ausgewählt wird.

Die bei der Nasszerkleinerung erhaltenen, je nach Arbeitsweise noch unterschiedliche Mengen an freien NCO-Gruppen aufweisenden TDI-Rückstandsschlacken werden entweder als sehr feinteilige Suspensionen, Pasten oder (nach Isolierung des Suspensionsmittels) Pulver in derselben Weise wie die durch Trockenzerkleinerung erhältlichen TDI-Rückstands-Pulver eingesetzt.

Für die Trockenmahlung wird unter 2 - 3 mm vorzerkleinerte und bevorzugt bei Temperaturen unter 50°C vorgetrocknete, nicht wesentlich über 20 Gew.-%, bevorzugt unter 10 Gew.-% Feuchtigkeit enthaltende TDI-Rückstandsschlacke eingesetzt. Bei der Wahl der Trockenzerkleinerungsmaschinen sind im wesentlichen nur die gewünschte Endfeinheit und Korngrössenverteilung, aber auch die Mahlkosten von vorrangiger Bedeutung. Die erfindungsgemäss eingesetzten Rückstandsschlacken sind im Vergleich zu Kunststoffen sehr hart und wegen ihres hohen Vernetzungsgrades in den üblichen Zerkleinerungsmaschinen ohne Kühlungsprobleme bei Temperaturen bis ca. 220 - 300°C mahlbar, ohne zu erweichen, was insbesondere zur Erzielung von sehr kleinen Korngrössen von besonderer Bedeutung ist.

Verwendung finden beispielsweise Stifts-, Kugel- oder Pralltellermühlen, ferner Luftstrommühlen wie Schlagkreuz-, Zahnkranz- oder Turbomühlen, aber besonders bevorzugt Dampfstrahl- oder Luftstrahlmühlen, weil in diesen die Zerkleinerung hauptsächlich durch gegenseitige Teilchenstösse, weniger durch Wandstösse, stattfindet und schon in einem Durchsatz Feinstkorngrössen erzielt werden. Selbstverständlich sind auch bei der Trockenzerkleinerung sowohl ein- als auch mehrstufige diskontinuierliche Mahlprozesse möglich.

Durch das Zerkleinern in der Nassphase oder im trockenen Zustand werden die in der Rückstandsschlacke inkludierten restlichen reaktiven Gruppen der obengenannten Art für die verschiedensten chemischen Reaktionen mit den Biomassen zugänglich gemacht.

Die Teilchengrösse des Rückstandspulvers soll unter 2 mm, zweckmässig unter 0,8 mm, bevorzugt unter 0,4 mm, besonders bevorzugt unter 0,1 mm, liegen, damit die Polyadditionsreaktionen mit den Biomassen möglichst quantitativ ablaufen können.

Weitere Einzelheiten hinsichtlich der Herstellung der im erfindungsgemässen Verfahren einzusetzenden TDI-Rückstandspulver sind der älteren deutschen Patentanmeldung P 2 846 815.7 (DE-A-2 846 815) zu entnehmen.

Dort werden auch im Deail mögliche Modifizierungsreaktionen an den TDI-Rückstandspulvern (z.B. mittels Carbonylverbindungen oder Verbindungen mit zerewitinow-aktiven Wasserstoffatomen) beschrieben, die man gegebenenfalls vor der erfindungsgemässen Verwendung der Pulver ausführen kann.

Das erfindungsgemässe Verfahren kann in verschiedenen Varianten ausgeführt werden, je nachdem, ob man von getrockneten oder von in Wasser dispergierten Biomassen ausgeht. Beim Arbeiten in wässriger Phase setzt man im allgemeinen Biomassen mit einem Feststoffgehalt von 0,3 bis 20 Gew.-%, vorzugsweise von 1 bis 15 Gew.-%, ein. Die wässrigen Überschussbelebtschlämme aus biologischen Kläranlagen haben in der Regel einen Feststoffgehalt von 0,3 bis 3 Gew.-%, insbesondere von 0,7 bis 1,5 Gew.-%. Die Menge des Isocyanats beträgt (bezogen auf die Summe aus Trockengewicht der Biomasse und Gewicht des Isocyanats) beim Arbeiten in wässriger Phase etwa 2 bis 95 Gew.-%, bevorzugt 3 bis 80 Gew.-%, während man insbesondere die TDI-Rückstandspulver bevorzugt in Mengen von etwa 20 bis 80 Gew.-% (jeweils bezogen auf die Summe aus Isocyanat und Trockengewicht der Biomassen) anwendet. Liegt hierbei das Isocyanat in einem stöchiometrischen Überschuss gegenüber den H-aciden Gruppen der Biomasse vor, so erhält man erfindungsgemäss Biomassen-Isocyanat-Polyadditionsprodukte mit freien NCO-Gruppen, was für manche Anwendungsgebiete der Verfahrensprodukte (z.B. als reaktive Füllstoffe) von besonderem Vorteil sein kann.

Die Denaturierung mit den oben beschriebenen TDI-Rückstandspulvern erfordert in der Regel etwas höhere Temperaturen, z.B. 70 bis 200°C, bevorzugt 90 bis 150°C. Im allgemeinen kann bei Mitverwendung eines Lösungsmittels die Reaktionstemperatur gegenüber der lösungsmittelfreien Arbeitsweise um ca. 20 - 30°C gesenkt werden.

Geht man erfindungsgemäss von in Wasser dispergierten Biomassen aus, dann ist es — vor allem bei gröberteiligem Material — vorteilhaft, ein organisches Lösungsmittel in einer Menge von 1 bis 30 Gew.-%, vorzugsweise 5 bis 10 Gew.-%, bezogen auf Dispersion mitzuverwenden, damit die Reaktion zwischen den im allgemeinen hydrophoben Isocyanaten und der wässrigen Biomasse erleichtert wird. Bevorzugt ist das organische Lösungsmittel wenigstens partiell mit Wasser mischbar. Geeignete Lösungsmittel für das erfindungsgemässe Verfahren, die gegebenenfalls auch gegenüber Isocyanaten reaktive Gruppen aufweisen können, sind z.B. Aceton, Methylethylketon, Methylalkohol, Ethylalkohol, Benzol, Toluol, Essigsäureethylester und deren Gemische.

Beim Arbeiten in wässriger Phase sind Temperaturen zwischen 80 und 130°C bevorzugt (gegebenenfalls kann auch unter Druck gearbeitet werden, beispielsweise bei einem Überdruck von 2 bis 100 bar). Der pH-Wert liegt im allgemeinen zwischen 1 und 10, vorzugsweise zwischen 4 und 8, und wird gegebenenfalls durch Zusatz von Säuren bzw. Alkali oder Ammoniak auf den gewünschten Bereich eingestellt. Durch hohe Temperaturen und niedrige pH-Werte wird während der Isocyanat-Polyadditionsreaktion die Plasmolyse, d.h. Schrumpfung des Protoplasmas und partielle Hydrolyse des Zellmaterials, begünstigt.

Die Polyadditionsreaktion in wässriger Phase kann erfindungsgemäss sowohl diskontinuierlich in üblichen Reaktionskesseln als auch, gegebenenfalls kombiniert dazu, kontinuierlich erfolgen, z.B. in Durchlaufmischern, wie sie beispielsweise in DE-C-2 513 815 (US-A-4 089 835) beschrieben werden oder in Mehrphasen-Reaktionsrohren gemäss DE-A-2 719 970 (US-A-4 119 613) und der dort zitierten Literatur. Die mittlere Verweilzeit des Reaktionsgemisches aus konzentrierter, wässriger Biomasse, Isocyanat und gegebenenfalls Lösungsmittel beträgt bei den kontinuierlichen Verfahren und Temperaturen nahe dem Siedepunkt vorzugsweise etwa 2 bis 20 Minuten, besonders bevorzugt 1 bis 5 Minuten. Von besonderem Interesse ist die Verwendung eines Mehrphasenströmungs-Reaktionsrohrs, da auf diese Weise auch eine fast quantitative Trocknung des Polyadditionsprodukts erreicht wird. Bei diskontinuierlichen Kesselansätzen wird durch die erfindungsgemässe Polyaddition Überschussbelebtschlamm aus biologisch arbeitenden Kläranlagen so weitgehend denaturiert und ausgeflockt, dass durch einfache Filtration die Verfahrensprodukte mit einem Feststoffgehalt von über 50 Gew.-% (auch ohne die sonst erforderlichen Filtrierhilfsmittel) isoliert werden können.

Verwendet man für das erfindungsgemässe Verfahren weitgehend wasserfreie Pulver von Biomassen, dann kann man davon augehen, dass der Zelltod bis auf einen kleinen Restgehalt lebender Zellen bereits eingetreten ist. Trotzdem haftet, wie bereits erwähnt, beispielsweise einem Belebtschlamm-Pulver ein uner-

trägIicher Geruch an. Dieser kann ebenso wie die latente Restaktivität mittels der erfindungsgemässen Isocyanat-Polyadditionsreaktion restlos beseitigt werden. Besser ist es, kurze Zeit (vorzugsweise, je nach Temperatur, 3 Minuten bis 3 Stunden) auf ca. 50 - 200°C, vorzugsweise 80 bis 150°C zu erhitzen. Auf diese Weise gewinnt man ein pulverförmiges, unlösliches Biomassen-Polyisocyanat. Die Umsetzung der Biotrockenmasse mit einer äquivalenten Menge oder einem Unterschuss an Isocyanaten führt zu NCO-freien, sterilisierten Biomassen-Polyadditionsprodukten. Auch bei dieser Verfahrensvariante liegt die eingesetzte Menge an Isocyanat (bezogen auf Gesamtmenge aus Biomasse und Isocyanat) bei 2 bis 95 Gew.-%, bevorzugt bei 3 bis 80 Gew.-%. Die Reaktion kann dabei entweder in Substanz oder in einem beliebigen der obengenannten organischen Lösungsmittel ablaufen (die Menge an Lösungsmittel beträgt dabei ca. 1 bis 50 Gew.-%, vorzugsweise 5 bis 20 Gew.-%, bezogen auf Reaktionsgemisch). Für das Arbeiten mit weitgehend wasserfreien Biomassen und einem flüssigen, organischen Lösungsmittel kommen wieder diskontinuierliche Kesselverfahren, jedoch auch kontinuierliche Verfahrensvarianten mittels Durchlaufmischern, Mehrphasen-Reaktionsrohren oder an sich bekannten Reaktionsschneckenmaschinen in Frage.

Von besonderer technischer Bedeutung, vor allem wenn das Reaktionsgemisch neben den Biomassen nur geringe Mengen an flüssigen Komponenten (z.B. Lösungsmittel oder flüssige Reaktionspartner für die Biomassen) enthält, und die oben näher beschriebenen pulverförmigen TDI-Rückstandschlacken als Isocyanatkomponente eingesetzt werden, sind zwei gegebenenfalls miteinander koppelbare Verfahrensweisen, nämlich erstens die an sich bekannte Schleuder- und Wirbeltechnik mit Hilfe von mechanisch wirksamen Mischern oder Mischwerkzeugen und/oder zweitens die Fliessbettechnik.

Zweckmässig verwendet man für die zuerst genannte Verfahrensweise im Handel befindliche heiz- bzw. kühlbare Mischer, bei denen in der Mischtrommel beispielsweise pflugscharähnliche Schaufeln um eine drehbare Achse und gegebenenfalls zusätzlich unabhängig bewegbare Messerköpfe montiert sind.

Wenn zunächst durch Laborversuche und anschliessend halbtechnische Versuche in 100-200 l-Mischapparaturen sichergestellt wurde, unter welchen Verfahrensbedingungen während der erfindungsgemässen Polyadditionsreaktion (Temperatur; Verweilzeit) ein weitgehend pulverartiger Zustand erhalten bleibt, ist der Einsatz grossvolumiger, gegebenenfalls hintereinander geschalteter Mischaggregate zur Produktion grosstechnischer Mengen an Biomassen-Isocyanat-Polyadditionsprodukten ohne grössere Schwierigkeiten möglich.

Für die Anwendung der zweiten Verfahrensweise, der Fliessbettechnik, ist der schwer berechenbare optimale Fluidisationszustand im Fliessbett, der bei gegebenen Feststoffdaten wie Dichte, Teilchengrössen und -verteilung und dem gewählten Strömungsmedium, beispielsweise Luft oder Stickstoff, wesentlich von der Differenz zwischen Lockerungsgeschwindigkeit und Geschwindigkeit des Strömungsmediums abhängt, in einfacher Weise durch einige Vorversuche in einem kleinen Laborfliessbett zu ermitteln, wobei man zweckmässig die gegebenenfalls vorgesehene Mitverwendung flüssiger oder gasförmiger Reaktionskomponenten schon in diese Vorversuche einbezieht.

Wenn die Rieselfähigkeit einer Schüttung während des Prozesses nicht ausreicht, und eine gewisse Klebrigkeit auftritt, kann in speziellen Fällen ein spezifisch schwereres, körniges Material, beispielsweise Quarzsand, mitverwendet und das Polyaddukt in einem Zyklon isoliert werden.

In Fliessbetten mit hohen Durchströmungsgeschwindigkeiten kann durch heftige Feststoffbewegung, gegebenenfalls auch während der erfindungsgemässen Reaktionen eine Feinzerkleinerung durchgeführt werden.

Wie schon erwähnt, kann man in einer speziellen Ausführungsform der vorliegenden Erfindung vor, nach oder gleichzeitig mit der erfindungsgemässen Isocyanat-Polyadditionsreaktion in den Biomassen auch Kondensationsreaktionen mit Carbonylverbindungen sowie gegebenenfalls Verbindungen, welche zur Aminoplast- und/oder Phenoplastbildung geeignet sind, ablaufen lassen. Derartige Kondensationsreaktionen sind nur insofern Gegenstand der vorliegenden Erfindung, als sie in der erwähnten Weise in Kombination mit Isocyanat-Polyadditionsreaktionen angewandt werden.

Die einfachste Modifizierung besteht darin, Formaldehyd auf die Biomassen einwirken zu lassen. Je nach pH erfolgt dabei zunächst lediglich eine Methylolierung der Biomassen oder, bevorzugt im stark sauren Bereich, eine Vernetzungsreaktion unter Ausbildung von Methylenbrücken. Neben einer zur Kondensation mit den Biomassen geeigneten Carbonylverbindung können, wie schon erwähnt, auch weitere kondensationsfähige Verbindungen zugegeben werden; darüber hinaus werden aber auch während der Isocyanat-Polyaddition selbst Verbindungen gebildet, die mit Carbonylverbindungen kondensieren können. Besonders interessant ist die Zugabe von Harnstoff oder die Ausbildung von kondensationsfähigen Harnstoffgruppen, ferner die Mitverwendung von Azulminsäure (siehe z.B. die Zusammenfassung von Th. Völker in Angewandte Chemie 1960, S. 379 - 384), die als polymere Blausäure zahlreiche Aminogruppen aufweist. Die auch gegenüber Isocyanaten reaktionsfähige Azulminsäure vermag darüber hinaus auch Schwermetallionen zu komplexieren. Erfindungsgemäss modifizierte Biomassen-Azulminsäure-Polyisocyanat-Polyadditionsprodukte sind daher als Pflanzennährstoffe besonders geeignet. Zudem erhöht sie den Stickstoffgehalt der Verfahrensprodukte beträchtlich.

Als Carbonylverbindungen, die gegebenenfalls bei der Durchführung des erfindungsgemässen Verfahrens als Reaktionskomponenten eingesetzt werden können, kommen alle üblichen Carbonylverbindungen mit ausreichend reaktiven Carbonylgruppen in Betracht. Hierzu gehören vorzugsweise Aldehyde und Ketone.

Besonders bevorzugte Aldehyde sind gesättigte, aliphatische, gegebenenfalls durch Halogen oder Hydroxy substituierte Mono-Aldehyde, wie Formaldehyd, Acetaldehyd, Butyraldehyd, Isobutyraldehyd, Pivalinaldehyd, Chloral, Hydroxyacetaldehyd, Hydroxypivalinaldehyd, Glycerinaldehyd, Hydroxyaldehyde, die in Formose-Zuckergemischen enthalten sind, und Hydroxyaldehyde, die aus anderen Aldehyden durch Aldol-Kondensationen entstehen. Besonders bevorzugte Aldehyde sind weiterhin ungesättigte aliphatische Aldehyde, wie Acrolein und Crotonaldehyd, ferner cycloaliphatische Aldehyde, wie Cyclohexanaldehyd, ausserdem aliphatische Dialdehyde, wie Glyoxal, Methylglyoxal, Glyoxalsulfat und Glutardialdehyd, darüber hinaus aromatische Aldehyde, wie Benzaldehyd, 4-Methylbenzaldehyd, Salicylaldehyd und Terephthaldialdehyd, sowie von Heterocyclen abgeleitete Aldehyde, wie Furfurol und Hydroxymethyl-furfurol.

Bevorzugt verwendbar sind auch «verkappte Aldehyde», also solche Verbindungen, die unter den Reaktionsbedingungen Aldehyde freisetzen oder wie Aldehyde reagieren. Speziell genannt seien in diesem Zusammenhang Paraformaldehyd, Trioxan, Chloralhydrat, Hexamethylentetramin und Halbacetale von Aldehyden, insbesondere von Formaldehyd, mit mono-, di- oder polyfunktionellen Alkoholen, wie Methanol, Äthanol, Butanol, Äthylenglykol und Diäthylenglykol.

Besonders bevorzugte Ketone sind Hydroxyaceton, Dihydroxyaceton, Methyläthylketon, Methylisobutylketon, Cyclohexanon, Acetophenon und Chinone, wie Benzochinon.

Verwendet werden können auch Mischungen aus Aldehyden und/oder Ketonen, wobei Mischungen aus Formaldehyd und anderen Aldehyden oder Ketonen besonders bevorzugt sind. Aus derartigen Mischungen von Formaldehyd mit solchen Aldehyden oder Ketonen, die α-ständige Wasserstoffatome besitzen, können durch Aldol-Kondensationen in situ Hydroxyaldehyde bzw. Hydroxyketone entstehen.

Die so entstehenden Hydroxyaldehyde und Polyhydroxyketone gehen im Masse ihrer Bildung, insbesondere im schwach bis stark alkalischen Bereich, zum Beispiel mit Harnstoff und vielen Aminoplastbildnern leicht Additionsreaktionen zu N-Alkylol-Verbindungen ein, die wiederum Kondensationspartner für die vorgenannten Biomassen darstellen.

Als Thiocarbonylverbindungen, die bei der Durchführung des erfindungsgemässen Verfahrens als Reaktionskomponenten eingesetzt werden können, kommen alle üblichen Thiocarbonylverbindungen mit ausreichend reaktiven Thiocarbonylgruppen in Betracht. Hierzu gehören vorzugsweise Thioaldehyde und Thioketone. Besonders bevorzugte Thioaldehyde und Thioketone sind solche, die sich von denjenigen Aldehyden und Ketonen ableiten, die bereits als besonders bevorzugt genannt wurden.

Bevorzugt verwendbar sind auch «verkappte Thioaldehyde», also solche Verbindungen, die unter den Reaktionsbedingungen Thioaldehyde freisetzen. Speziell genannt sei der trimere Thioformaldehyd — das Trithian —, der bei erhöhter Temperatur in Gegenwart von Säuren in Thioformaldehyd zerfällt.

Als Carbonylverbindungen, die mit niedermolekularen, nicht kondensierten N-Alkylolverbindungen im Dissoziationsgleichgewicht stehen, kommen vorzugsweise einfache Aldehyde, insbesondere Formaldehyd in Betracht, der mit den entsprechenden N-Methylolverbindungen im Gleichgewicht steht. Zu solchen N-Methylolverbindungen gehören insbesondere N-Methylolharnstoff, N,N'-Dimethylol-harnstoff, methyloliertes Dicyandiamid, methyloliertes Oxamid, N-Methylol-thioharnstoff, N,N'-Dimethylol-thioharnstoff und methylolierte Melamine, wie Hexamethylolmelamin und Trishydroxymethyl-melamin der Konstitution

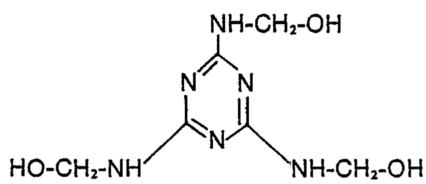

Weiterhin genannt sei Monomethylol-äthylenharnstoff der Konstitution

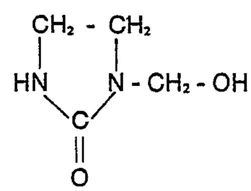

Monomethylol-äthylenthioharnstoff der Konstitution

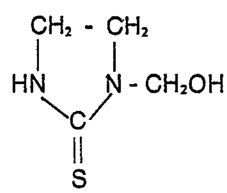

und Tetramethylolacetylen-diharnstoff der Konstitution

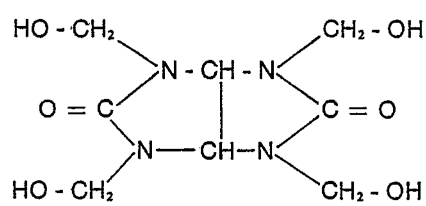

In Betracht kommen ferner Alkylol-Verbindungen, die sich von einfachen Aldehyden ableiten,

bevorzugt von solchen mit bis zu 5 Kohlenstoffatomen.

Insbesondere kommen bei der Durchführung des erfindungsgemässen Verfahrens als Carbonylverbindungen die folgenden Stoffe in Frage: Formaldehyd, Acetaldehyd, Isobutyraldehyd, Crotonaldehyd, Glyoxal, Furfurol, Hydroxymethylfurfurol, Salicylaldehyd sowie deren Halbacetale, ausserdem Polymere des Formaldehyds, wie Paraformaldehyd und Trioxan, ferner Hexamethylentetramin und auch Thioaldehyde, wie Thioformaldehyd. Als nicht kondensierte (niedermolekulare) N-Alkylolverbindungen kommen bei der Durchführung des erfindungsgemässen Verfahrens insbesondere in Betracht: N-Methylolharnstoff, Dimethylolharnstoff, Trimethylolmelamin, Hexamethylolmelamin, Monomethylol-äthylenharnstoff, Monomethylol-äthylenthioharnstoff und Tetramethylolacetylen-diharnstoff.

Wie schon erwähnt, können beim erfindungsgemässen Verfahren zwecks Modifizierung der Biomassen auch Aminoplastbildner eingesetzt werden. Unter Aminoplastbildnern sind hierbei alle diejenigen Stickstoff-Verbindungen zu verstehen, die in der Lage sind, mit reaktionsfähigen Carbonyl-Verbindungen N-Oligo- und N-Polykondensationsprodukte zu bilden.

Hierzu gehören Stickstoffverbindungen, wie Harnstoffe, z.B. der Harnstoff selbst, Acetylenharnstoff, Dimethylacetylenharnstoff und N-Methylenharnstoff, ferner Thioharnstoffe, wie der unsubstituierte Thioharnstoff, weiterhin Diharnstoffe, wie Hexamethylendiharnstoff, Tetramethylendiharnstoff und Äthylendiharnstoff, ausserdem Polyharnstoffe, wie sie durch Umsetzung von aliphatischen, cycloaliphatischen bzw. araliphatischen Di- oder Triisocyanaten oder auch Biuret-polyisocyanaten mit Ammoniak oder primären Aminen erhalten werden, darüber hinaus Polycarbonsäureamide, wie Oxalsäurediamid, Bernsteinsäurediamid und Adipinsäurediamid, ferner Mono-, Di- und Polyurethane, wie beispielsweise die Umsetzungsprodukte von aliphatischen, cycloaliphatischen, araliphatischen und aromatischen Mono- oder Bischlorameisensäureestern mit Ammoniak oder primären Aminen, ausserdem Biurete, Melamine, wie das Melamin selbst, Amidine, wie Dicyandiamidin, Guanidine, wie Aminoguanidin, weiterhin Guanazole, Guanamine, Cyanamid, Dicyandiamid, primäre Monoamine, sekundäre Monoamine, Arylamine, Ammoniak, Diamine, Triamine, Hydrazine und Carbonsäurehydrazide, wie Hydrazodicarbonamid, Carbacinsäureester und Hydrazodicarbonsäureester, und ausserdem ähnliche zur Aminoplastbildung befähigte Stickstoffverbindungen, vorzugsweise die den vorgenannten Stickstoffverbindungen entsprechenden, N-Alkylolgruppen, vorzugsweise N-Methylolgruppen, aufweisenden Derivate und die entsprechenden $C_1$-$C_4$ Alkyläther dieser N-Alkylol-Derivate.

Ferner kommen als Aminoplastbildner vorzugsweise auch höhermolekulare $\alpha,\bar{\omega}$-Di-harnstoffe in Betracht, deren N-Methylol-Derivate und N-Methylolalkyläther, ferner $\alpha,\bar{\omega}$-Bis-alkoxymethylurethane, welche zwischen den $\alpha,\bar{\omega}$-ständigen funktionellen Gruppen Polyäther-, Polythioäther-, Polyacetal-, Polyester-, Polyesteramid- oder Polycarbonatreste vom Durchschnittsmolekulargewicht 400 bis 10 000 und gegebenenfalls zusätzliche Urethan- oder substituierte Harnstoffgruppen aufweisen. Besonders bevorzugt sind hierbei als höhermolekulare zur Aminoplastbildung befähigte Stickstoffverbindungen wasserlösliche oder in Wasser dispergierbare Verbindungen, z.B. Verbindungen, welche zwischen den $\alpha,\bar{\omega}$-ständigen funktionellen Urethan- oder Harnstoffgruppen Polyäthylenoxidreste od. Reste von Mischpolymerisaten des Äthylenoxids mit Propylenoxid bzw. Tetrahydrofuran oder von wasserlöslichen Polyacetalen, hergestellt aus Di-, Tri- oder Tetraäthylenglykol und Formaldehyd, aufweisen.

Diese als Ausgangsverbindungen geeigneten Aminoplastbildner sind bekannt oder lassen sich nach im Prinzip bekannten Methoden herstellen [vgl. Houben-Weyl «Methoden der organischen Chemie», Band XIV, Teil 2 (1963), Seiten 319 bis 402, Georg Thieme-Verlag, Stuttgart].

Bei der Durchführung des erfindungsgemässen Verfahrens kommen als Aminoplastbildner vorzugsweise auch «modifizierte Aminoplastbildner» in Betracht. Hierunter sind Aminoplastbildner zu verstehen, die zusätzliche leicht einbaubare Gruppen enthalten. Genannt seien z.B. Verbindungen, die rasch und leicht durch Mischkondensation einbaufähig sind. Hierzu gehören vorzugsweise Polyurethane und Polyharnstoffe mit $NH_2$-Endgruppen, Polyamide aus Poly-(β-alanin) mit Molgewichten bis 2000, N-Methylolmethyläther von Polycaprolactam, Polythiolactame, Polypeptide aus N-Carboxy-α-aminocarbonsäuren, niedermolekulare Polyamide aus aliphatischen Dicarbonsäuren und Diaminen, Polyamide aus cycloaliphatischen Komponenten und aromatischen Komponenten, Polyamide mit O- bzw. S- oder N- als Heteroatome, Polyesteramide, Mischkondensate, die neben Amidgruppen noch Ester-, Urethan- oder Harnstoffgruppen enthalten, äthoxylierte und propoxylierte Mono- und Polyamide, Polyhydrazide und Polyaminotriazole, Polysulfonamide, Formaldehyd-Mischkondensate mit Harnstoff, Melamin und Dicyandiamid, niedermolekulare Anilin-Formaldehyd-Kondensate, Sulfonsäureamide, Mono- und Dinitrile, Acrylnitril, Urotropin, Hexahydrotriazine aus primären Aminen und Formaldehyd, Schiff'sche Basen und Ketimine oder Polyketimine, wie z.B. solche aus einem Mol Hexamethylendiamin und 2 Mol Cyclohexanon, Polyadditionsprodukte und Polykondensationsprodukte des Melamins und anderer Aminoheterocyclen mit Aldehyden und Alkoholen, Polyadditions- und Polykondensationsprodukte von Nitrilen mit Aldehyden, Umsetzungsprodukte von phosphoriger Säure und Dialkylphosphiten mit Carbonylverbindungen und Aminen bzw. Polyaminen. Ferner kommen als zur Aminoplastbildung befähigte Verbindungen in diesem Zusammenhang auch diejenigen Verbindungen in Frage, die in der DE-A-2 324 134 auf den Seiten 7 bis 12 angeführt sind.

Zu den modifizierten Aminoplastbildnern, die bei dem erfindungsgemässen Verfahren eingesetzt werden können, gehören weiterhin N-Alkylolverbindungen und insbesondere N-Methylolverbindungen, die zum Teil mit Polyhydroxylverbindungen veräthert sind.

Der Anteil von Alkoholen bzw. Polyalkoholen in diesen Produkten kann je nach Komponente bis zu 60 Gew.-%, bezogen auf die Summe der Prozente an Stickstoff-Verbindungen und Alkoholen, betragen.

Bei der Durchführung des erfindungsgemässen Verfahrens kommen als Aminoplastbildner insbesondere unter anderem die folgenden Stoffe in Frage: Harnstoff, Thioharnstoff, Diharnstoffe, wie z.B. Hexamethylendiharnstoff, Tetramethylendiharnstoff, Äthylenharnstoff, Acetylenharnstoff, Dimethylacetylenharnstoff, Oxalsäurediamid, Bernsteinsäurediamid, Adipinsäurediamid, Mono- oder Bishydrazide, wie Hydrazodicarbonamid, Carbazinsäure-methyl- und äthylester, Hydrazodicarbonsäureester, Mono- und insbesondere Diurethane, wie beispielsweise die Umsetzungsprodukte von aliphatischen, cycloaliphatischen, araliphatischen und aromatischen Mono- oder Bis-chlorameisensäureestern mit Ammoniak und primären Aminen, ferner Anilin, Melamin, Dicyandiamid, Cyanamid, Aminoguanidin, Dicyandiamidin, Guanamine, Guanazole, ferner Polyharnstoffe und Polybiurete, wie sie durch Umsetzung von aliphatischen, cycloaliphatischen, araliphatischen Di- oder Triisocyanaten, wie auch Biuretpolyisocyanaten mit einem Überschuss an Ammoniak oder primären Aminen erhalten werden.

Im übrigen kommen bei der Durchführung des erfindungsgemässen Verfahrens als Aminoplastbildner auch nahezu fehlerstellenfreie Azulminsäuren, fehlerstellenhaltige sogenannte modifizierte Azulminsäuren, durch Kondensation mit Carbonylverbindungen stabilisierte Azulminsäuren, ferner durch Kondensation mit Carbonylverbindungen und Aminoplastbildnern bzw. deren niedermolekularen Kondensationsprodukten stabilisierte Azulminsäuren, sowie Metallsalzkomplexe der vorgenannten Azulminsäuren in Frage. Diese Stoffe werden bei dem erfindungsgemässen Verfahren bevorzugt zusammen mit anderen Aminoplastbildnern, insbesondere Harnstoff, eingesetzt.

Diese verschiedenen Azulminsäuren sind bekannt und werden z.B. in Houben-Weyl, Methoden der Organ. Chemie (1952), Band 8, Seite 261; in Angewandte Chemie 72, (1960), Seiten 379 - 384; den DE-Patentschriften 662 338 und 949 600 sowie den DE-A-2 806 019, 2 806 020, 2 844 641 und 2 844 642 eingehend beschrieben.

Für das erfindungsgemässe Verfahren geeignete Phenoplastbildner sind die an sich bekannten Phenole und deren Derivate wie z.B. Phenol, Kresol, Bisphenol A, Nitrophenol, Brenzkatechin, Hydrochinon und Naphtholsulfonsäure. Weitere als Modifizierungsmittel geeignete Aminoplast- und Phenoplastmonomere werden in den DE-A-2 324 134, 2 713 198, 2 738 532 und 2 844 641 beschrieben.

Im übrigen können im erfindungsgemässen Verfahren auch Biomassen eingesetzt werden, welche bereits nach der Verfahrensweise der älteren europäischen Patentanmeldung 10 243 denaturiert worden sind.

Derartige Biomassen werden erhalten, indem man sie

- in einer ersten Reaktionsphase, gegebenenfalls unter hydrolytischem Abbau bzw. unter Denaturierung der in den Biomassen vorhandenen Zellwände in wässrigem Medium mit Carbonylverbindungen, Thiocarbonylverbindungen und/oder Carbonylverbindungen, die mit niedermolekularen, nicht kondensierten N-Alkylolverbindungen im Dissoziationsgleichgewicht stehen, gegebenenfalls in Gegenwart eines Katalysators sowie gegebenenfalls in Gegenwart von Zusatzstoffen kondensiert,
- dann in einer zweiten Reaktionsphase die nicht umgesetzten Carbonylverbindungen, Thiocarbonylverbindungen und/oder Carbonylverbindungen, die mit niedermolekularen, nicht kondensierten N-Alkylolverbindungen im Gleichgewicht stehen, in wässrigem Medium mit Aminoplastbildnern, die gegebenenfalls N-Alkylolgruppen enthalten, bzw. mit Phenoplastbildnern, gegebenenfalls in Gegenwart eines Katalysators und gegebenenfalls in Gegenwart von Kettenabbrechern sowie gegebenenfalls in Gegenwart von Zusatzstoffen umsetzt und
- die so entstehenden modifizierten Biomassen gegebenenfalls anschliessend von noch enthaltenen unerwünschten Stoffen befreit und/oder einer Nachbehandlung unterwirft.

Die erfindungsgemäss hergestellten Polyadditionsprodukte aus denaturierten Biomassen, Isocyanaten und gegebenenfalls Aminoplast- bzw. Phenoplastbildnern können noch nachbehandelt werden, indem man sie, gegebenenfalls in Gegenwart von Verdünnungsmitteln, wie z.B. wasserfreien organischen Lösungsmitteln, bei Temperaturen zwischen 0°C und 200°C, vorzugsweise zwischen 10°C und 140°C, besonders bevorzugt zwischen 30°C und 120°C, mit den verschiedensten Reagenzien behandelt. Hierbei treten im wesentlichen an der Oberfläche der Verfahrensprodukte chemische Reaktionen auf, so dass man an der Oberfläche chemisch modifizierte Produkte erhält.

Für eine derartige chemische Modifizierung der Oberfläche der nach dem erfindungsgemässen Verfahren herstellbaren Polyadditionsprodukte kommen vorzugsweise in Betracht

- die Behandlung mit Harnstoffschmelzen;
- die Behandlung mit Acylierungsmitteln, wie Ameisensäure, Essigsäureanhydrid oder gemischten Säureanhydriden aus Essigsäure und Ölsäure (vorzugsweise in Gegenwart von Natrium- oder Kaliumacetat); cyclischen Säure-Anhydriden, wie Maleinsäureanhydrid, Phthalsäureanhydrid oder Hexahydrophthalsäureanhydrid; Schmelzen von Dicarbonsäuren, wie Adipinsäure, Phthalsäure, Hexahydrophthalsäure oder Trimellitsäure; anorganischen Säurechloriden, wie Chlorcyan, Phosgen, Thionylchlorid, Schwefelchloriden, Phosphoroxychlorid, Phosphorpentachlorid, Siliciumtetrachlorid, Antimontrichlorid oder Titantetrachlorid; organischen Säurechloriden, wie Acetylchlorid, Benzoylchlorid, Chlorameisensäureestern, Benzolsulfonsäurechloriden, Phosphorsäureesterchloriden, Chlormethansulfochlorid oder Cyanursäurechlorid;

- die Behandlung mit Alkylierungsmitteln, wie Dimethylsulfat, Methyljodid oder Methylbromid, Dichloräthan, Glykolchlorhydrin, Chloressigsäureäthylester, Dichloressigsäureäthylester, Chloracetaldehyd-diäthylacetal, Allylchlorid, Benzylchlorid, Trichlormethylisocyaniddichlorid oder anderen Isocyaniddichloriden;
- die Behandlung mit ε-Caprolactam, ε-Caprolacton, Hydroxypivalinsäurelacton, cyclischen 6gliedrigen oder 8gliedrigen Siloxanen, Azalactamen wie sie aus der DE-A-2 035 800 bekannt sind, Glykolcarbonat, Äthylenoxid, Propylenoxid, Butylenoxid, Styroloxid, Epichlorhydrin, Butyrolacton, Valerolacton, Oxazolidinen, Oxazolinen, Imidazolidinen, Isatosäureanhydrid oder Leuchsschen Anhydriden aus Aminosäuren und Phosgen;
- die Behandlung mit Acrylnitril oder anderen Vinylmonomeren, wie Acrylsäure, Methacrylsäure bzw. deren Methyl-, Äthyl-, β-Hydroxyäthyl- oder Propylestern;
- die Behandlung mit Hydroxy-alkanphosphonsäureestern bzw. den zugrundeliegenden Säuren, insbesondere mit Hydroxymethyl-phosphonsäureestern bzw. der freien Hydroxymethyl-phosphonsäure;
- die Behandlung mit Chlormethylalkoxysilanen;
- die Behandlung mit den verschiedensten Mono- oder Polynitrilen, bevorzugt Hydroxymethylnitril, unter den Bedingungen der durch Hydroxyanionen katalysierten Thorpe-Reaktion;
- die Behandlung mit Polyisocyanaten der oben genannten Art in Gegenwart von aus der Polyurethanchemie an sich bekannten, gegenüber Isocyanaten reaktiven Verbindungen (insbesondere Polyolen mit einem Molekulargewicht zwischen 62 und 500). Auf diese Weise kann man die denaturierte Biomasse mit einer Polyurethanhülle umgeben, ohne dass die pulverförmige Konsistenz des Materials verloren geht. Ein ähnlicher Effekt wird erreicht, wenn man die oben erwähnten, noch freie NCC-Gruppen enthaltenden denaturierten Biomassen mit Polyolen nachbehandelt bzw. carbodiimidisiert.

Als Nachbehandlungsreagenzien seien ausserdem erwähnt: Natriumhydroxid, Kaliumhydroxid, Calciumhydroxid, Natriumsulfid, Rongalit, Ammoniumpolysulfide, Diäthylphosphit und Dimethylphosphit.

Bei diesen Nachbehandlungsreaktionen können auch die verschiedensten Mischpolymerisationen oder Polymerisationen von Vinylmonomeren durchgeführt werden, wobei die Biomassen-Mischkondensate durch die entstehenden Polymeren umhüllt bzw. mikroverkapselt werden. Hierbei können selbstverständlich die «Hüllmaterialien» auch in einem grossen Überschuss eingesetzt werden.

Derartig modifizierte Biomassen können — ebenso wie die weiter oben erwähnten polyurethanumhüllten Produkte — direkt, d.h. ohne Zusatz weiterer Bindemittel, unter Erwärmen zu Formkörpern verpresst werden.

In speziellen Fällen können die oben unter dem Sammelbegriff «Nachbehandlung» diskutierten Modifizierungsreaktionen an den Biomassen auch schon vor bzw. simultan mit der erfindungsgemässen Polyadditionsreaktion ausgeführt werden. Es ist auch möglich, im Anschluss an die Polyadditionsreaktion auf den Verfahrensprodukten Polymethylenharnstoffe, Polyalkylidenharnstoffe und andere schwer- oder unlösliche Verbindungen, z.B. hochvernetzte Aminoplastkondensate, zu erzeugen, die infolge ihrer Unlöslichkeit praktisch keine kovalente Verknüpfungen zur Biomasse aufweisen. Derartige Mischungen, in denen die Menge des nicht kovalent gebundenen Anteils an Aminoplastkondensaten bzw. Phenoplastkondensaten beliebig variiert sein kann, stellen insbesondere für den Fall der Beladung mit Polymethylenthioharnstoffen, vernetzten Polymethylenmelaminpulvern, Harnstoff-Hydrazodicarbonamid-Formaldehydkondensaten und Dicyandiamid- bzw. Oxamid-Kondensaten ausserordentlich interessante Flammschutzmittel für die verschiedensten Kunststoffe dar.

Von Interesse ist auch die nachträgliche Beladung der Verfahrensprodukte mit schwerlöslichem Melaminphosphat, schwerlöslichem Harnstoffoxalat, Harnstoffnitrat oder schwerlöslichem Ammoniummagnesiumphosphat. Auch die Zugabe von Tonerdehydraten, Aluminiumoxiden, Alumosilikaten, Calciumcarbonat, von Quarzmehl sowie die Zugabe linearer oder vernetzter Polymethylenharnstoffe, von pulvrigen Melamin-Formaldehyd-Kondensaten, Harnstoff-Hydrazodicarbonamid-Kondensaten und hochmolekularen Polyammoniumpolyphosphaten ist von Bedeutung. Die dabei entstehenden Produkte eignen sich hervorragend als Flammschutzmittel für Kunststoffe.

Weiterhin kommen bei dem erfindungsgemässen Verfahren, insbesondere dann, wenn Azulminsäuren mitverwendet werden, als bevorzugte Zusatzstoffe auch Zucker, wie Rohrzucker und andere keine freien Aldehydgruppen aufweisenden Zucker, oder auch aus Formaldehyd hergestellte Formose-Zuckergemische in Frage. Diese verschiedensten Zuckerarten können in Kanälen und Poren der Azulminsäure fixiert werden. Darüber hinaus können die verschiedenen Zucker auch in Form ihrer meist schwer löslichen Calcium-Komplexe auf den Mischkondensaten aufziehen.

Ferner ist es stets dann, wenn die erfindungsgemässen Polyaddukte Azulminsäuren enthalten, möglich, die Produkte nach ihrer Herstellung gleichzeitig mit Ammoniak und Kohlendioxid zu begasen. Dabei dringen Ammoniak und Kohlendioxid als kleine Moleküle in beträchtlichem Masse in das Azulminsäuregerüst ein.

Neben den eingehend beschriebenen reaktiven Aminoplast-, Phenolplast- und Vinylmonomeren bzw. sonstigen reaktiven niedermolekularen Verbindungen können den Biomassen im erfindungsgemässen Verfahren die verschiedensten Füll- und Zusatzstoffe beigemischt werden, z.B. organische Naturstoffe und daraus gewonnene Produkte, anorganische Naturstoffe und daraus gewonnene Produkte synthetische organische Produkte, synthetische anorganische Produkte und/oder gemischt organisch-anorganische Produkte.

Vorzugsweise in Frage kommende organische Naturstoffe und daraus gewonnene Produkte sind hierbei Holzpulver oder Späne, Ligninpulver, Ligninsulfonsäuren, ammonifizierte Ligninsulfonsäuren, Humus, Huminsäuren, ammonifizierte Huminsäuren, Torf, Proteine und deren Abbauprodukte, z.B. Polypeptide, wie Wolle und Gelatine, Fischmehl und Knochenmehl, ferner Pektine, Polysaccharide, wie Stärke und Cellulose, Hemicellulosen, homogenisierte Materialien pflanzlichen und tierischen Ursprungs, Aktivkohle sowie Aschen, die durch Verbrennung organischer, durch Photosynthese gebildeter Stoffe oder üblicher Brennstoffe erhalten wurden.

Als anorganische Naturstoffe und daraus gewonnene Produkte kommen vorzugsweise in Betracht Silikate, wie Aluminiumsilikate, Calciumsilikate, Magnesiumsilikate und Alkalisilikate, Kieselsäuren, insbesondere disperse Kieselsäuren, Kieselgele, weiterhin Tonmineralien, Glimmer, Carbonate wie Calciumcarbonat, Phosphorit und Phosphate wie Calciumphosphat und Ammoniummagnesiumphosphat sowie Sulfate wie Calciumsulfat.

Als synthetische organische Produkte kommen vorzugsweise neben natürlichen oder Synthesekautschuken, Polyamiden und Epoxidharzen die schon ausführlich beschriebenen Aminoplast- und Phenoplastharze in Frage.

Besonders geeignete Zusatzstoffe sind auch pulverförmige TDI-Rückstandsschlacken der weiter oben beschriebenen Art, deren NCO-Gruppen durch Umsetzung mit Wasser oder anderen H-aciden Verbindungen quantitativ abreagiert wurden (derartig modifizierte TDI-Destillationsrückstände sind ebenfalls in den schon erwähnten DE-A-2 846 809 und DE-A-2 846 815 beschrieben). Auch wenn sie NCO-frei sind, enthalten derartige Pulver noch zahlreiche reaktive Gruppen (z.B. Harnstoff-, Urethan-, Carbodiimid- und/oder Uretdiongruppen), welche an den beim erfindungsgemässen Verfahren ablaufenden Polyadditions- und Polykondensationsreaktionen teilnehmen können.

Als synthetische anorganische Produkte, die vorzugsweise in Frage kommen, seien genannt Düngemittel, wie Superphosphat, Thomasschlakke, Rhenaniaphosphat, Phosphorit, Kalkstickstoff, Kalkammonsalpeter, Leunasalpeter, Kaliumphosphate, Kaliumnitrat und Ammoniumnitrat, ferner Pigmente, wie Eisenoxide und Titandioxide, insbesondere jedoch die anorganischen Primärschlämme aus biologischen Kläranlagen.

Die erfindungsgemäss hergestellten Polyadditionsprodukte aus denaturierten Biomassen, Isocyanaten sowie gegebenenfalls Zusatzstoffen eignen sich insbesondere dann, wenn sie keine freien NCO-Gruppen enthalten, hervorragend als agrochemische Mittel (Agrochemikalien). Hierunter sind Mittel zu verstehen, die für die verschiedenartigsten Verwendungen in der Landwirtschaft und im Gartenbau in Frage kommen.

So eignen sich die erfindungsgemäss hergestellten Stoffe z.B. als Düngemittel sowohl zur Versorgung von Pflanzen mit Makronährstoffen als auch zur Versorgung von Pflanzen mit Mikronährstoffen; insbesondere eignen sie sich als Stickstoff-Düngemittel mit Langzeitwirkung. Von besonderem Interesse sind hierbei solche erfindungsgemäss verwendbaren Stoffe, die von Pflanzen benötigte Ionen, wie Ammonium-Ionen, Lithium-, Natrium-, Kalium-, Beryllium-, Magnesium-, Calcium-, Strontium-, Barium-, Aluminium-, Zink-, Mangan-, Nickel-, Kobalt- und Eisen-Ionen enthalten.

Weiterhin von besonderem Interesse als Düngemittel sind solche erfindungsgemäss verwendbaren Stoffe, die Anionen, wie Chlorid, Nitrat, Sulfat und/oder Phosphat enthalten.

Besonders bevorzugt als Düngemittel sind solche erfindungsgemässen Stoffe, die mehrere der vorgenannten Ionensorten nebeneinander enthalten. Beispielhaft genannt seien Stoffe, die sowohl Kalium- und/oder Ammonium-Ionen als auch Nitrat- und/oder Phosphat-Ionen enthalten.

Von besonderem Interesse als Düngemittel sind ausserdem solche erfindungsgemässen Stoffe, die gegebenenfalls neben Nährstoffionen auch die oben im Detail beschriebenen organischen Zusatzstoffe enthalten.

Bevorzugt verwendbar als Düngemittel sind ausserdem solche erfindungsgemässen Stoffe, die gegebenenfalls neben Nährstoffionen auch handelsübliche Düngemittel enthalten. Speziell genannt seien in diesem Zusammenhang als derartige handelsübliche Düngemittel Superphosphat, Thomasschlacke, Rhenaniaphosphat, Phosphorit, Kalkstickstoff, Kalkammonsalpeter, Leunasalpeter, Kaliumphosphate, Kaliumnitrat und Ammoniumnitrat, ferner Harnstoff-Formaldehyd-Kondensate, Harnstoff-Crotonaldehyd-Kondensate, Harnstoff-Isobutyraldehyd-Kondensate sowie Kondensate aus Dicyandiamid, Melamin oder Oxamid mit Aldehyden, wie Formaldehyd, Acetaldehyd, Crotonaldehyd oder Isobutyraldehyd.

Bevorzugt verwendbar als Düngemittel sind auch solche erfindungsgemässen Stoffe, die gegebenenfalls neben Nährstoffen auch biologisch aktive Gartenerde enthalten.

Werden solche anmeldungsgemässen Produkte als Düngemittel eingesetzt, bei deren Herstellung Schwermetallsalze enthaltende Biomassen verwendet wurden, so ist es erforderlich, bei der Herstellung derartiger Produkte Azulminsäuren, Thioharnstoff oder andere stark komplexierend wirkende Verbindungen als Aminoplastbildner zuzufügen. Auf diese Weise werden in den Biomassen enthaltene Schwermetallionen, wie z.B. Ionen des Bleis, Kupfers, Quecksilbers, Cadmiums oder des Zinks so fest gebunden, dass keine Pflanzenschädigungen auftreten.

Besonders bevorzugt als Düngemittel verwendbar sind jedoch Produkte auf Basis schwermetallfreier Biomassen, wie sie z.B. bei Fermentationsprozessen der Arzneimittel-, Enzym-, Lebensmittel- und Genussmittel-Industrie anfallen, ferner schwermetallfreie Biomassen aus biologisch oder vollbiologisch arbeitenden Kläranlagen für industrielle und kommunale Abwässer.

Bevorzugt als Düngemittel verwendbar sind auch solche anmeldungsgemässen Biomassen-Mischkondensate, die innerhalb der ankondensierten oder beigemengten Polymethylenharnstoff-Gruppen durch Mitverwendung von Isobutyraldehyd auch Segmente der Struktur

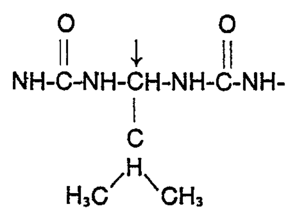

als verknüpfende Bauelemente enthalten. Die durch den Pfeil gekennzeichnete Stelle ist hydrolytisch wesentlich anfälliger als methylenverknüpfte Harnstoff-Segmente. Die betreffenden Stoffe lassen sich sehr gut als Düngemittel mit rascher und über lange Zeit gleichmässiger Stickstoffabgabe verwenden.

Bevorzugt verwendbar als Düngemittel sind auch solche anmeldungsgemässen Stoffe, bei deren Herstellung Azulminsäuren der verschiedensten Art eingesetzt wurden. Derartige Produkte zeichnen sich wegen der vielfältigen chemischen Reaktionsfähigkeit und der Sorptionsfähigkeit der Azulminsäuren durch eine besonders hohe Variabilität im Aufbau aus. So können z.B. relativ grosse Mengen an Säuren, bevorzugt Phosphorsäure und Salpetersäure, gebunden werden. Im Überschuss vorhandene Säuren können z.B. durch Begasung mit Ammoniak neutralisiert werden. Solche Produkte sind in der Lage, den Pflanzen nebeneinander sowohl organisch als auch anorganisch gebundenen Stickstoff zur Verfügung zu stellen.

Diejenigen Produkte, die nach ihrer Herstellung noch Aldehyde, wie z.B. Formaldehyd enthalten, werden vor dem Einsatz als Stickstoff-Düngemittel zweckmässigerweise mit Aminen oder Ammoniak behandelt. Formaldehyd wird z.B. durch Ammoniak in Hexamethylentetramin überführt. Letzteres ist ein gut wirksamer Stickstoffdünger.

Werden bei der Herstellung der erfindungsgemäss modifizierten Biomassen Azulminsäuren (Rohazulminsäuren, modifizierte Azulminsäuren und/oder stabilisierte Azulminsäuren) mitverwendet, so können normalerweise sehr leicht wasserlösliche Zellinhaltsstoffe der Biomassen wie Polysaccharide, wasserdispergierbare oder lösliche Glykolipide, Lipoproteide, abgebaute Proteine, Nucleinbasen, abgebaute aber nicht kondensierte Nucleinsäuren, vollständig auf Azulminsäuren adsorbiert werden, so dass diese für die Humifizierung und für die Pflanzenernährung wertvollen Stoffe nicht in das Abwasser geraten, sondern den Pflanzen als Stickstoffdünger zur Verfügung stehen.

Diejenigen isocyanatfreien Verfahrensprodukte, bei deren Herstellung schwermetallsalzfreie, proteinreiche Biomassen und gegebenenfalls physiologisch verträgliche Zusatzstoffe verwendet werden, lassen sich als Tierfutter-Zusatzmittel verwenden.

Weiterhin eignen sich die erfindungsgemäss hergestellten modifizierten schwermetallfreien Biomassen als Bodenverbesserungsmittel. Bevorzugt verwendbar zu diesem Zweck sind solche anmeldungsgemässen Produkte, die Holzpulver bzw. pulverisiertes pflanzliches Material enthalten. Bevorzugt verwendbar als Bodenverbesserungsmittel sind auch solche erfindungsgemäss verwendbaren modifizierten Biomassen, bei deren Herstellung Azulminsäuren mitverwendet wurden.

Diejenigen erfindungsgemäss verwendbaren modifizierten Biomassen, die fehlerstellenreiche Azulminsäuren in gebundener Form enthalten, besitzen bis zu einem gewissen Grade Polyelektrolytcharakter und können im Boden als Stickstoff-Düngemittel mit Ionenaustauscher-Eigenschaften fungieren. Hierbei werden die von den Pflanzen benötigten Ionen, z.B. Kalium- und/oder Ammonium-Ionen an das Erdreich bzw. an das Substrat abgegeben, während andere Ionen gebunden werden.

Infolge der hohen Sorptionsfähigkeit und des guten Komplexbildungsvermögens können Azulminsäuren bzw. andere zur Komplexbildung neigende Stoffe enthaltende erfindungsgemäss verwendbare modifizierte Biomassen auch zur Fixierung von Schadstoffen im Boden verwendet werden. So ist es z.B. möglich, im Boden vorhandene unerwünschte Schwermetallionen, wie z.B. Ionen des Bleis und des Quecksilbers mit Hilfe der erfindungsgemäss verwendbaren, Azulminsäure enthaltenden Stoffe so fest zu binden, dass Pflanzenschädigungen nicht mehr zu befürchten sind. Ferner können auch Ölverunreinigungen, Überdosierungen an Pflanzenschutzmitteln oder zu hohe Salzkonzentrationen in Substraten durch Zugabe derartiger erfindungsgemäss verwendbarer Stoffe beseitigt werden.

Erfindungsgemäss verwendbare Stoffe, die neben anderen Pflanzennährstoffen auch Torf enthalten, lassen sich durch Zusatz von Bindemitteln, wie Stärke, abgebauten Cellulosen, Alginaten und Pektinstoffen, in technisch einfacher Weise zur Herstellung von Torfpresstöpfen für den gärtnerischen Betrieb verwenden. Hierbei ist es zweckmässig, dass der Volumenanteil an Weiss- und Schwarztorf im Substrat etwa 1 : 1 beträgt.

Erfindungsgemäss verwendbare modifizierte Biomassen, die neben Stickstoff und anderen Pflanzennährstoffen etwa 20 bis 40 Gew.-% an Torf enthalten, eignen sich auch gut zur Abdeckung für Böden und Substrate sowie Saatreihen, da durch die dunkle Farbe der erfindungsgemässen Stoffe ein gutes erdiges Aussehen gewährleistet ist, Bodenverkrustungen verhindert werden und raschere Keimung in Saatreihen bewirkt wird.

Erfindungsgemäss verwendbare Stoffe, die Torf enthalten, eignen sich auch zur Verhinderung oder Abschwächung von Geruchsbildungen bei Verwesungsprozessen.

Erfindungsgemäss verwendbare Stoffe, die neben anderen Planzennährstoffen auch Torf enthalten, lassen sich durch Zusatz von Stärkeklebern, Hemicellulosen oder Alginaten in geformte, luftdurchlässige und Feuchtigkeit haltende Materialien überführen, die als Packmaterialien für den Pflanzentransport geeignet sind.

Die erfindungsgemäss verwendbaren Stoffe können als solche oder in ihren Formulierungen zur Versorgung von Pflanzen mit Stickstoff und gegebenenfalls anderen Nährstoffen bzw. als Bodenverbesserungsmittel eingesetzt werden.

Hierbei können die erfindungsgemäss verwendbaren Stoffe in die üblichen Formulierungen übergeführt werden, wie Emulsionen, Spritzpulver, Suspensionen, Pulver, Stäubemittel, Schäume, Pasten, Granulate, Suspensions-Emulsions-Konzentrate, Saatgutpuder, Wirkstoff-imprägnierte Natur- und synthetische Stoffe oder Feinstverkapselungen in polymeren Stoffen und in Hüllmassen für Saagut.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeguende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Stoffe können in den Formulierungen in Michung mit anderen Düngemitteln oder pestiziden Wirkstoffen vorliegen.

Die Anwendung geschieht nach den in der Landwirtschaft und im Gartenbau üblichen Methoden, also z.B. durch direktes Einbringen in den Boden, durch Giessen, Verspritzen, Versprühen, Verstreuen, Verstäuben usw. Als spezielle Applikationsarten seien genannt: Wurzelapplikation, Blattapplikation, Stamminjektion und Rindenapplikation. Bei der Wurzelapplikation kann der Dünger entweder mit dem Kultursubstrat vermischt oder auch in Bodenfurchen eingebracht werden. Ferner ist es möglich, den Dünger mit Hilfe einer Düngerlanze sowie durch Schlag- oder Bohrlöcher in den tieferen Wurzelbereich einzuführen.

Die eingesetzte Menge an erfindungsgemässen Stoffen kann in grösseren Bereichen variiert werden. Bei einer Anwedung als Dünger bzw. Bodenverbesserungsmittel hängt sie im wesentlichen ab von der Bodenart sowie vom Nährstoffbedarf der jeweiligen Pflanzen. Im allgemeinen liegen die Aufwandmengen an Wirkstoff zwischen 0,1 und 200 kg/ha, vorzugsweise zwischen 1 und 100 kg/ha. Werden die erfindungsgemässen Stoffe für andere Zwecke verwendet, wie zur Abdeckung von Substraten, zur Herstellung von Packmaterialien für Pflanzen, zum Schutz von Pflanzen oder Pflanzenteilen, zur Herstellung von Torfpresstöpfen oder zur Bindung von unerwünschten Geruchsstoffen, so ist

die eingesetzte Menge an Wirkstoff dem jeweiligen Bedarf angepasst.

Die erfindungsgemäss hergestellten, getrockneten und pulverisierten Polyadditionsprodukte aus denaturierten Biomassen, Isocyanaten sowie gegebenenfalls Zusatzstoffen eignen sich — insbesondere auch, wenn sie noch freie Isocyanatgruppen aufweisen — hervorragend als reaktiver Füllstoff für die verschiedensten Polyadditions-, Polykondensations- und/oder Polymerisationsreaktionen, z.B. in einem Anteil von 2-95 Gew.-%, bevorzugt 10-70 Gew.-%, besonders bevorzugt 15-40 Gew.-%, bezogen auf Gesamtmenge an modifiziertem Kunststoff. (Die Verwendung als Füllstoff für Polyurethankunststoffe fällt unter eine Parallelanmeldung der Anmelderin und soll von der vorliegenden Erfindung nicht mitumfasst werden).

Wie schon erwähnt, können bestimmte umhüllte denaturierte Biomassenpulver direkt (d.h. ohne weiteres Bindemittel) als hitzeverformbare Pressmassen eingesetzt werden.

Bevorzugt werden die erfindungsgemäss erhaltenen Pulver in Aminoplast- und Phenoplastharze als Füllstoff eingearbeitet, wobei sie vorteilhafterweise schon bei der an sich bekannten Herstellung dieser Harze aus Carbonylverbindungen (insbesondere Formaldehyd) und Aminoplast- bzw. Phenoplastmonomeren (vorzugsweise Harnstoff, Melamin und/oder Phenol) anwesend sind und über ihre zahlreichen reaktiven Gruppierungen in das sich bildende Polymere chemisch eingebaut werden. In diesem Zusammenhang geeignete Carbonylverbindungen bzw. Aminoplast- und Phenoplastmonomere werden z.B. in den DE-A-2 324 134, 2 639 254 u. 2 713 198 beschrieben.

Auch bei der Herstellung von Epoxidharzen können die erfindungsgemäss aufgearbeiteten Biomassen als reaktive Komponente mitverwendet werden.

In der modifizierten Biomasse vorhandene Isocyanatgruppen können sowohl mit den in technischen Epoxidharzen stets vorhandenen Hydroxylgruppen, als auch bei höheren Temperaturen, bevorzugt über 160°C, mit den Epoxidgruppen unter Bildung von Oxazolidonringen reagieren.

Bevorzugt werden die pulverisierten modifizierten Biomassen in flüssige Diepoxide bei Raumtemperatur oder erhöhter Temperatur homogen eingemischt und gegebenenfalls in Anwesenheit eines Härters, beispielsweise einer Aminoverbindung, Dicarbonsäure oder eines Dicarbonsäureanhydrids, unter den an sich bekannten Verfahrensbedingungen zur Reaktion gebracht. In vielen Fällen können (insbesondere, wie oben ausgeführt, bei Härtungstemperaturen über 100°C) die polyfunktionellen Biomassen partiell gleichzeitig sowohl mit dem Epoxidharz als auch mit dem Härter während der Epoxid-Polyaddition reagieren, so dass der reaktionsfähige Füllstoff durch Hauptvalenzbindungen in das ausgehärtete Giessharz eingebaut ist.

Die in Mengen von insgesamt bis ca. 50 Gew.-%, bezogen auf Endprodukt, zugesetzten Biomassen vermindern insbesondere die Brennbarkeit der auf Epoxidbasis hergestellen Harze und vermindern ausserdem den Schrumpf. Bei grossen Giesslingen tritt während der Härtung im Inneren eine geringere Temperatursteigerung auf als bei Füllstoff-freien Giesslingen.

Die erfindungsgemäss aufgearbeiteten Biomassen können jedoch auch als reaktiver Füllstoff bei der Herstellung von Cyanatharzen, beispielsweise aus den Ausgangsverbindungen, wie sie in der DE-A-2 260 487 beschrieben werden, mitverwendet werden.

Insbesondere Biomassen, in welche gegebenenfalls copolymerisierbare, ungesättigte Gruppierungen eingeführt wurden, können auch mit Vorteil bei der an sich bekannten Herstellung von Kunststoffen durch Polymerisation oder Copolymerisation olefinisch ungesättigte Gruppen aufweisender Monomerer eingesetzt werden. Als derartige Monomere seien beispielhaft Acrylnitril, Styrol, Butadien, Acrylsäure, Methacrylsäure, Vinylchlorid, Vinylacetat sowie ungesättigte Polyester genannt.

Die Polymerisationsreaktionen werden (in Gegenwart der sehr feinteiligen Biomassen) bevorzugt in einem flüssigen Medium, beispielsweise in Wasser oder einem organischen Lösungsmittel durchgeführt.

Die erfindungsgemäss hergestellten, gegebenenfalls noch freie NCO-Gruppen enthaltenden modifizierten Biomassen können durch Polymerisationsreaktionen in einfacher Weise umhüllt werden, indem man monomere oder oligomere Vinylverbindungen in Anwesenheit der feinstzerkleinerten Biomassen, gebebenenfalls in einem gegenüber Isocyanatgruppen inerten Lösungsmittel, (co)polymerisiert.

Besondere Bedeutung besitzen die erfindungsgemäss aufgearbeiteten Biomassen auch als Bindemittel oder als Füllstoff bei der Herstellung von Platten oder Formteilen in Heisspressen durch Bindung von lignocellulosehaltigen Fasern, Spänen oder Lagen. Als weitere Bindemittel dienen dabei vorzugsweise die zu diesem Zweck an sich bekannten Kondensationsprodukte des Formaldehyds mit Harnstoff, Melamin oder Phenol, insbesondere in Form ihrer wässrigen Lösungen oder Dispersionen. Aus DE-A-1 669 759 und DE-B-1 653 169 ist es bereits bekannt, neben oder anstelle von derartigen Bindemitteln bei der Herstellung von Presswerkstoffen auf Basis lignocellulosehaltiger pflanzlicher Rohstoffe auch Polyisocyanate mitzuverwenden.

Es wurde nunmehr gefunden, dass sich für diesen Zweck die erfindungsgemäss modifizierten Biomassen (vor allem, wenn sie noch freie NCO-Gruppen besitzen oder weitere Isocyanate mitverwendet werden) hervorragend eignen. Sie werden dabei in einer Menge von 2-90, bevorzugt 10-60 Gew.-%, bezogen auf Gesamtgewicht des Formkörpers, eingesetzt.

Geeignete lignocellulosehaltige Rohstoffe, welche auf diese Weise gebunden werden können, sind beispielsweise Holz, Rinde, Kork, Bagasse, Stroh, Flachs, Bambus, Alfagras, Reisschalen, Sisal- und Kokosfasern. Das Material kann dabei in Form von Granulaten, Spänen, Fasern oder Mehl vorliegen und einen Wassergehalt von 0 bis 35 Gew.-%, vorzugsweise von 5 bis 25 Gew.-%, aufweisen. Es wird gegebenenfalls mit einem Polyisocyanat und/oder einem Formaldehydharz in einer Menge von 1 bis 50, vorzugsweise 5 bis 20 Gew.-% (berechnet als Feststoff, bezogen auf Gesamtgewicht des Formkörpers) sowie der oben angegebenen Menge an modifizierter Biomasse versetzt und — im allgemeinen unter Einwirkung von Druck und Hitze — zu Platten oder Formkörpern verpresst.

In der selben Weise können auch mehrlagige Platten oder Formteile aus Furnieren, Papieren oder Geweben hergestellt werden. Auch mehrschichtige Platten oder Formteile aus Furnieren und Streifen-, Stab- oder Stäbchenmittellagen, sogenannte Tischlerplatten, können auf diesem Weg hergestellt werden, indem man die Furniere wie oben beschrieben mit der modifizierten Biomasse sowie gegebenenfalls dem konventionellen Bindemittel behandelt und anschliessend mit den Mittellagen — in der Regel bei erhöhter Temperatur und erhöhtem Druck — verpresst. Vorzugsweise werden dabei Temperaturen von 100 bis 250°C, besonders bevorzugt 130 bis 200°C, eingehalten. Der Anfangspressdruck liegt vorzugsweise zwischen 5 und 150 bar; im Laufe des Pressvorgangs fällt dann der Druck meist bis gegen 0 ab. Selbstverständlich können auch die an sich bekannten organischen oder anorganischen Schutzmittel gegen Pilzbefall, Insekten oder Feuereinwirkung in einer Menge von ca. 0,05 bis 30 Gew.-%, vorzugsweise 0,5 bis 20 Gew.-%, mitverwendet werden.

Gegenstand der Erfindung ist auch somit die Verwendung der erfindungsgemäss modifizierten Biomassen, sowie gegebenenfalls zusätzlicher konventioneller Formaldehyd-Harze als Bindemittel in Mengen von 2 bis 90 Gew.-%, bezogen auf Gesamtgewicht des Formkörpers, zur Herstellung von Platten oder Formteilen durch Heissverpressen lignocellulosehaltiger Rohstoffe.

Die erfindungsgemäss aufgearbeiteten Biomassen können auch ganz allgemein Lacken und Überzügen der verschiedensten Art als reaktiver Füllstoff beigemengt werden (ca. 2 bis 70 Gew.-%, bevorzugt 5 bis 40 Gew.-%, bezogen auf Gesamtfeststoffgehalt). Beispiele hierfür sind Dach- oder Bodenbeläge, Fugenverguss- und Spachtelmassen der an sich bekannten Art, gegebenenfalls unter Mitverwendung von Bitumen- bzw. Teermassen. Eine weitere Verwendungsmöglichkeit besteht in der Modifizierung thermoplastischer Kunststoffe. Die Biomassen werden dabei mit dem Thermoplasten in einer Menge von 3 bis 200 Gew.-%, bevorzugt 10 bis 100 Gew.-%, bezogen auf Thermoplast, mittels an sich bekannter Techniken (z.B. durch Co-Extrusion) vermischt und gegebenenfalls thermoplastisch, z.B. durch Spritzgiessen oder Verpressen, verformt.

Werkstoffe dieser Art können beispielsweise zur Herstellung von Konstruktionteilen oder Möbeln dienen.

Die folgenden Beispiele dienen der Erläuterung der vorliegenden Erfindung; wenn nicht anders vermerkt, sind Mengenangaben als Gewichtsteile bzw. Gewichtsprozente zu verstehen.

In den folgenden Patentbeispielen 1 - 5 wurde ein 7 - 12%iger wässriger Belebtüberschussschlamm («BS») als Biomasse eingesetzt, der durch Vermehrung von Mikroorganismen, insbesondere Bakterien, Pilzen und Protozoen, aus industriellen und kommunalen Abwässern in einer vollbiologisch arbeitenden Kläranlage entstanden war und durch Zentrifugieren eines ursprünglich ca. 1 Gew.-% organische Substanz enthaltenden Klärschlamms erhalten wurde. Die eingesetzten Belebtüberschussschlamm-Partien hatten einen gelartigen Charakter, waren nicht filtrierbar und verbreiteten bereits im frischen, biologisch noch aktiven Zustand einen unerträglichen Geruch. Beim Stehen innerhalb weniger Tage faulte die unbehandelte Biomasse unter Gasentwicklung. Der Stickstoffgehalt der Trockenmasse lag bei 7,8 bis 8,5 Gew.-%, der Glühverlust betrug 81 - 87 Gew.-%.

In der Praxis werden diese überschüssigen wässrigen Klärschlämme in der Kläranlage, um sie filtrierbar zu machen, mit der gleichen bis doppelten Menge, bezogen auf Feststoff, anorganischer Primärschlämme gemischt, auf Filterpressen zu einem ca. 50%igen Presskuchen abgedrückt und zu Mülldeponien verfrachtet.

Die Patentbeispiele 1 - 5 erläutern die Aufarbeitung der Biomassen durch Isocyanatpolyaddition in wässriger oder organisch-wässriger, disperser Phase. Rezepturen und Versuchsergebnisse sind in Tabelle 1 zusammengestellt.

Tabelle 1

| Beispiel | Biomasse Konz. 1 | Typ 2 | pH 3 | Isocyanat 4 | Lösungsmittel 5 | Trockenmasse Gew.-% 6 | Stickstoff 7 | Geruch 8 |
|---|---|---|---|---|---|---|---|---|
| 1 | 11,4 | BSA | 6,8 | 100 T 80-R1 | 5 Ac | 35 | 12,5 | frei |
| 2 | 12,1 | BSD | 1,7 | 100 T 80-R2 | 20 Ac | 51 | 12,6 | sehr gering |
| 3 | 11,4 | BSA | 6,8 | 100 T 80-R2 | 10 To | 45 | 12,7 | frei |
| 4 | 11,4 | BSA | 3,0 | 200 T 80-R2 | 5 Ac | 48 | 14,2 | frei |
| 5 | 12,1 | BSD | 1,7 | 100 T 80-R3 | 10 To | 39 | 12,8 | frei |

**Erläuterungen zu Tabelle 1**

Spalte 1: Konzentration des verwendeten wässrigen Belebtüberschussschlamms (BS) in Gew.-%.

Spalte 2: Typ des Belebtüberschussschlamms:
BSA = biologisch voll aktiver, d.h. frisch vom Klärbecken als 1%iges Sediment abgezogener, zentrifugierter BS. Viskosität bei 25°C: 580 mPaS.
BSD = biologisch desaktivierter BS. Der BS wurde durch 2stündiges Kochen unter Rückfluss mit 2,5 Gew.-% einer 38%igen wässrigen Formaldehydlösung bei pH 6,5 - 7 denaturiert, wobei eine partielle Hydrolyse der Biomasse erfolgt.
BSA und BSD sind nicht filtrierbare Ausgangsmaterialien.

Spalte 3: pH = pH-Wert während der Isocyanatpolyaddition; die Endprodukte werden jeweils auf pH 6 - 7 eingestellt.

Spalte 4: Gewichtsteile Isocyanat, bezogen auf 100 Teile BSA(D)-Feststoff.

Bei den Isocyanaten T80-R1, R2 und R3 handelt es sich um granulierte Isocyanat-Rückstandsschlacken aus einer grosstechnischen 2,4-/2,6-Toluylendiisocyanatproduktion (Isomerenverhältnis: 80 : 20), welche gemäss der DE-A-2 846 815 durch Denaturierung der NCO-haltigen, ca. 150-200°C heissen, zähflüssigen, teerartigen Sumpfphase (Destillationsrückstand) mit der ca. 4- bis 5fachen Menge Wasser erhalten wurden und — je nach Trockentemperatur (40 bis 70°C) — den angegebenen Restisocyanatgehalt aufweisen, der bei 50°C in Aceton bestimmt wurde.

T80-R1 NCO-Gehalt: 14,2%; Teilchengrösse 20-250 µm
T80-R2 NCO-Gehalt: 9,9%; Teilchengrösse 30-500 µm
T80-R3 NCO-Gehalt: 5,0%; Teilchengrösse 100-800 µm

Spalte 5: Gewichtsteile Lösungsmittel, bezogen auf 100 Teile des eingesetzten, wässrigen Belebtüberschussschlamms
Ac = Aceton; Tol = Toluol.

Spalte 6: Gewichtsprozent Trockenmasse im Filterkuchen nach Absaugen des Wassers und Lösungsmittels.

Spalte 7: Stickstoffgehalt der Trockenmasse in Gewichtsprozent.

Spalte 8: Geruchsqualifikation des erfindungsgemäss hergestellten Produktes.

**Vergleichsversuche** (ohne Isocyanat-Zugabe)

Setzt man die in Spalte 2 genannten Belebtschlamm-Partien den erfindungsgemässen Verfahrens- und Reaktionsbedingungen aus, wie sie nachfolgend beschrieben werden, unterlässt jedoch die Zugabe der genannten Isocyanate, so bleibt der unerträgliche Geruch bei BSD erhalten, bei BSA wird er beim Lagern unter starker Gasentwicklung (u.a. Abspaltung von Schwefelwasserstoff) beträchtlich gesteigert, so dass beispielsweise eine Ausbringung als Pflanzennährstoff unmöglich ist.

Verfahrens- und Reaktionsbedingungen für die Patentbeispiele 1 - 5 und die Vergleichsversuche ohne Isocyanatpolyaddition

In einer Kesselrührapparatur aus VA-Stahl mit Rückflusskühler wird der Belebtüberschussschlamm bei Raumtemperatur vorgelegt und die in Tabelle 1 genannte Menge Isocyanat eingerührt. Das Lösungsmittel kann entweder vor der Zugabe mit dem Isocyanat vereinigt oder getrennt eingetragen werden. Der Kesselinhalt wird bis zum Kochen unter Rückfluss erhitzt und 1 - 3 Stunden bei der Siedetemperatur gehalten. Falls in Tabelle 1 ein saurer pH-Wert angegeben ist, wird dieser mit Schwefelsäure, bevorzugt vor der Isocyanatzugabe, eingestellt. Sobald kein freies Isocyanat mehr nachgewiesen werden kann, wird mit Natriumtriphosphat oder

Alkalilauge neutralisiert und nach dem Abkühlen auf einer üblichen Drucknutsche oder Filterpresse abgedrückt. Der feuchte Filterkuchen wird, falls eine Verringerung des Wassergehaltes erwünscht ist, in einem Umluftheizschrank oder durch Ausbreiten an der Luft getrocknet, bis der gewünschte Wassergehalt erreicht wird. Für die Verwendung als geruchsfrei bleibender Langzeitdünger für Gärtnereien und in der Landwirtschaft wird ein Wassergehalt von ca. 10 - 40 Gewichtsprozent und eine Korngrösse von 1 - 4 mm bevorzugt.

Beispiel 6

2000 Teile 12,1%iger, desaktivierter, wässriger Belebtschlamm, 10 Teile Emulgator aus 1 Mol Oleylalkohol und 400 Mol Äthylenoxid und 242 Teile des pulverisierten Toluylendiisocyanat-Rückstandsteers T80-R2 werden innig vermischt und in einem Druckgefäss 1 Stunde auf 145°C erhitzt. Nach dem Abkühlen wird abgenutscht. Der feuchte Filterkuchen hat einen Feststoff-Gehalt von 38%. Der Stickstoffgehalt der geruchlosen Trockenmasse beträgt 12,7%.

In dem folgenden Patentbeispiel 7 wird die erfindungsgemässe Herstellung von Biomassen-Polyadditions-Polykondensationsprodukten durch simultan ablaufende Isocyanatpolyadditions- und Aminoplastkondensationsreaktionen in wässrigorganischer Phase beschrieben.

Beispiel 7

Rezeptur:

880 Gewichtsteile 11,4%iger, wässriger, biologisch vollaktiver Klärschlamm, mit Schwefelsäure auf pH 2,1 eingestellt,
100 Gewichtsteile pulverisierte Toluylendiisocyanat-Rückstandsschlacke T80-R2 mit einem NCO-Gehalt von 9,9%,
40 Gewichtsteile 30%ige, wässrige Formaldehyd-Lösung,
30 Gewichtsteile Harnstoff und
100 Gewichtsteile Toluol

Reaktionsbedingungen:

Die genannten Komponenten werden in einer Kesselrührapparatur, die mit einem Rückflusskühler ausgestattet ist, bei Raumtemperatur vereinigt und unter Rühren bis zur Siedetemperatur erhitzt. Man lässt ca. 2 - 3 Stunden unter Rückfluss kochen, bis kein freies Isocyanat mehr nachgewiesen werden kann. Dann wird mit Calciumhydroxidlösung neutralisiert, bis der pH bei 6,5 - 7 konstant bleibt. Nach dem Abkühlen wird in einer Drucknutsche bei 0,5 - 2 bar abgedrückt. Der geruchlose Nutschkuchen hat einen Feststoffgehalt von 47%. In der Trockenmasse sind 18,4% Stickstoff enthalten.

In den nachfolgenden Patentbeispielen 8 - 14 werden als Biomasse bei ca. 110°C getrocknete, übel riechende Belebtüberschussschlamm-Pulver aus vollbiologisch arbeitenden, industriellen und kommunalen Kläranlagen eingesetzt, wie sie üblicherweise in Deponien gelagert oder in seltenen Fällen verbrannt werden. Im feucht gewordenen Zustand reaktivieren sich diese Belebtschlamm-Pulver biologisch innerhalb weniger Tage und faulen unter zunehmender starker Geruchsbelästigung weiter aus.

Die Biomassen dieses Typs werden in den folgenden Beispielen erfindungsgemäss durch Isocyanatpolyaddition in überwiegend organischer, disperser Phase oder nur mit organischen Lösungsmitteln benetzt denaturiert. Rezepturen und Versuchsergebnisse sind in Tabelle 2 zusammengestellt.

Tabelle 2

| Beispiel | Verfahren | Isocyanat | Lösungsmittel | Zusätze | Trockenmasse | | |
|---|---|---|---|---|---|---|---|
| | | | | | Stickstoff | Geruch | NCO-Gehalt |
| 1 | 2 | 3 | 4 | 5 | 6 | 7 | 8 |
| 8 | I | 200 T 80 R2 | 100 Tol | — | 11,3 | frei | 0 |
| 9 | I | 100 T 80 R3 | 80 Tol | — | 13,0 | frei | 0 |
| 10 | I | 100 T 80 R2 | 80 Ac | 100 Az | 23,4 | frei | 0 |
| 11 | II | 100 T 80 R2 | 20 Tol | — | 12,8 | frei | 0 |
| 12 | II | 100 T 80 R2 | 25 Ac | 20 Uro | 15,5 | frei | 0 |
| 13 | III | 100 T 80 R3 | — | — | 12,8 | frei | 0 |
| 14 | I | 100 T 80 R2 | 100 Ac | 100 ABS | 9,7 | | 0 |

Erläuterungen zu Tabelle 2

Spalte 1: Laufende Nummer des Patentbeispiels

Spalte 2: Verfahrensmethode (siehe nachfolgende Beschreibung).

Spalte 3: Isocyanat-Rückstandsschlacken wie in Tabelle 1 erläutert (Gewichtsteile, bezogen auf 100 Gewichtsteile Belebtüberschussschlamm-Pulver).

Spalte 4: Lösungsmittel in Gewichtsteilen, bezogen auf Gesamtfeststoff. Ac = Aceton; Tol = Toluol.

Spalte 5: Zusätze (Gewichtsteile, bezogen auf 100 Teile Belebtüberschussschlamm-Pulver)

Az = Azulminsäure, nach DE-A-2 806 019 durch Polymerisation von Blausäure hergestellt (Teilchengrösse: 10-200 µm),

Uro = Urotropin, in Wasser (mit katalytischer Menge Schwefelsäure) 30%ig gelöst,

ABS = Pfropfcopolymerisat aus Butadien-Styrol-Acrylnitril; als 33%ige wässrige Dispersion zugegeben.

Spalte 6: Stickstoffgehalt der Trockenmasse in Gewichtsprozent.

Spalte 7: Geruchsqualifikation des erfindungsgemäss hergestellten Produktes.

Spalte 8: NCO-Gehalt der getrockneten Verfahrensprodukte.

Verfahrens- und Reaktionsbedingungen für die Patentbeispiele 8 - 14

Verfahrensmethode I

Die in einer Kesselrührapparatur mit Rückflusskühler bei Raumtemperatur vereinigten Komponenten werden 2 - 4 Stunden unter Rühren bis zum Sieden erhitzt, bis der NCO-Gehalt Null ist.

Das Lösungsmittel wird entweder vollständig unter Rühren abdestilliert, wobei man gegen Ende die Temperatur um 30 - 40°C steigert und die pulverförmigen Verfahrensprodukte aus dem Kessel durch Überdruck austrägt oder pneumatisch absaugt oder man lässt die im organischen Medium dispergierten Biomassen-Polyadditionsprodukte abkühlen, filtriert das Dispersionsmittel ab und trocknet anschliessend.

Verfahrensmethode II

a) Diskontinuierliche Variante

Die in der Tabelle 2 genannten Komponenten werden in einem Kneter mit der angegebenen geringen Menge Aceton oder Toluol benetzt und in einem Druckgefäss 30 - 90 Minuten auf 110 bis 140°C unter dem Eigendruck erhitzt. Nach Beendigung der Isocyanat-Polyaddition lässt man bis nahe zur Siedetemperatur des Lösungsmittels abkühlen und destilliert dieses quantitativ ab.

Die Verfahrensprodukte werden erkaltet als geruchlose, sterile Pulver isoliert, die auch beim Befeuchten mit Wasser und Langzeitlagerung nicht mehr ausfaulen.

b) Kontinuierliche Variante (erfindungsgemäss bevorzugt)

Mit dem gleichen Ergebnis kann man die Reaktionskomponenten, bevorzugt mit Toluol als Benetzungsmittel, in einen zweiwelligen Ausdampfextruder eindosieren und bei einer Verweilzeit von 10 - 30 Minuten die Isocyanat-Polyaddition bei 140 - 170°C ablaufen lassen. Das Benetzungsmittel wird vor dem Austritt der pulverförmigen Verfahrensprodukte aus der Schneckenmaschine destillativ vollständig zurückgewonnen.

Verfahrensmethode III (besonders bevorzugt)

Die pulverförmigen Ausgangsverbindungen werden kontinuierlich in ein Fliessbett eingetragen und bei einer Temperatur von 145 - 180°C und einer mittleren Verweilzeit von 10 - 20 Minuten zur Reaktion gebracht. Das geruchlose Belebtschlamm-Rückstandspolyisocyanat-Polyadditionsprodukt wird kontinuierlich durch Zwangsförderung von unten nach oben dem Reaktor entnommen.

**Patentansprüche**

1. Verfahren zur Herstellung denaturierter Polyadditionsprodukte aus Biomassen und Isocyanaten, gegebenenfalls unter Zusatz weiterer polymerbildender Stoffe und nachträglicher Temperaturbehandlung, dadurch gekennzeichnet, dass man

A) 5 bis 98 Gew.-%, bezogen auf A) + B), einer Biomasse auf Basis von Mikroorganismen bzw. deren Folge- und Zersetzungsprodukten mit

B) 95 bis 2 Gew.-%, bezogen auf A) + B), bei der grosstechnischen Isocyanatherstellung anfallender Destillationsrückstände, gegebenenfalls in Gegenwart von

C) Wasser und/oder einem organischen Lösungsmittel sowie gegebenenfalls in Gegenwart von

D) organischen und/oder anorganischen Zusatzstoffen,

bei Temperaturen oberhalb von 50°C unter vollständiger Denaturierung von Komponente A) umsetzt.

2. Verfahren nach Anspruch 1, dadurch gekennzeichnet, dass als Biomasse wässrige oder getrocknete, gegebenenfalls bereits der aeroben oder anaeroben Faulung unterworfene Klärschlämme aus industriellen bzw. kommunalen Kläranlagen eingesetzt werden.

3. Verfahren nach Anspruch 1, dadurch gekennzeichnet, dass als Biomasse Hefen aus technischen Fermentationsprozessen zur Herstellung von Alkohol, Essigsäure, Milchsäure, Zitronensäure oder Weinsäure oder zur Proteinherstellung aus Erdöl, Paraffin, Methan oder Methanol eingesetzt werden.

4. Verfahren nach Anspruch 1, dadurch gekennzeichnet, dass Biomassen der Penicillin-, Tetracyclin- oder Sisomycin-Herstellung eingesetzt werden.

5. Verfahren nach Anspruch 1 bis 4, dadurch gekennzeichnet, dass als Komponente B) ein im wesentlichen monomerenfreier, vernetzter, in inerten organischen Lösungsmitteln unlöslicher, nicht unzersetzt schmelzbarer Destillationsrückstand mit einem NCO-Gehalt von 1 bis 15 Gew.-

% und einer Teilchengrösse < 2 mm eingesetzt wird, welcher durch (a) Entfernung des monomeren Toluylendiisocyanats aus dem rohen Phosgenierungsprodukt von Toluylendiaminen, (b) gegebenenfalls Einrühren des Rückstands in Wasser, (c) Vermahlen und gegebenenfalls (d) gleichzeitige und/oder anschliessende Modifizierungsreaktionen mit gegenüber den funktionellen Gruppen des Rückstands reaktiven Verbindungen erhalten wird, und dass die Denaturierungsreaktion bei Temperaturen zwischen 70 und 200°C ausgeführt wird.

6. Verfahren nach Anspruch 1 bis 5, dadurch gekennzeichnet, dass man Destillationsrückstände B) verwendet, die durch Einrühren des Rückstandes in Wasser und anschliessende Mahlung auf eine Teilchengrösse unter 0,8 mm gebracht werden.

7. Verfahren nach Anspruch 1 bis 6, dadurch gekennzeichnet, dass die Destillationsrückstände B) in Mengen von 80 bis 10 Gew.-%, bezogen auf A) + B) verwendet werden.

8. Verfahren nach Anspruch 1 bis 7, dadurch gekennzeichnet, dass die Denaturierungsreaktion, im wesentlichen in Abwesenheit flüssiger Komponenten, in einem Wirbel- oder Fliessbett ausgeführt wird.

9. Verfahren nach Anspruch 1 bis 8, dadurch gekennzeichnet, dass vor, nach oder simultan mit der Isocyanatpolyadditionsreaktion eine Kondensationsreaktion der Biomasse mit Carbonylverbindungen und gegebenenfalls weiteren zur Aminoplast- und/oder Phenoplastbildung befähigten Verbindungen stattfindet.

10. Verfahren nach Anspruch 9, dadurch gekennzeichnet, dass als Carbonylverbindung Formaldehyd verwendet wird.

11. Verfahren nach Anspruch 1 bis 10, dadurch gekennzeichnet, dass organische und/oder anorganische Füllstoffe mitverwendet werden.

12. Verwendung der nach Anspruch 1 bis 11 hergestellten denaturierten Polyadditionsprodukte als Pflanzennährstoff.

13. Verwendung der nach Anspruch 1 bis 11 hergestellten denaturierten Polyadditionsprodukte als hitzeverformbare Pressmassen und als reaktiver Füllstoff bei der Herstellung von Kunststoffen durch Polyadditions-, Polykondensations- und/oder Polymerisationsreaktionen.

14. Verwendung der gemäss Anspruch 1 bis 11 hergestellten denaturierten Polyadditionsprodukte, gegebenenfalls zusammen mit konventionellen Formaldehyd-Harzen als Bindemittel in Mengen von 2 bis 90 Gew.-%, bezogen auf das Gesamtgewicht des Formkörpers, bei der Herstellung von Platten oder Formteilen durch Heissverpressen lignocellulosehaltiger Rohstoffe.

**Claims**

1. Process for the production of denatured polyaddition products of biomasses and isocyanates, optionally with the addition of other polymer-forming compounds followed by thermal treatment, characterised in that

A) 5 to 98% by weight, based an A) + B), of a biomass based in micro-organisms or derivatives and decomposition products thereof,

are reacted with

B) 95 to 2% by weight, based on A) + B), of distillation residues accumulating in the commercial production of isocyanates,

optionally in the presence of

C) water and/or an organic solvent,

and optionally in the presence of

D) organic and/or inorganic additives,

at temperatures higher than 50°C with complete denaturing of component A).

2. Process according to claim 1, characterised in that aqueous or dried purified sludges from idustrial or communal purification plants which may have been subjected to aerobic or anaerobic digestion are used as the biomass.

3. Process according to claim 1, characterised in that yeasts from industrial fermentation processes for the production of alcohol, acetic acid, lactic acid, citric acid or tartaric acid or for the production of proteins from petroleum, paraffin, methane or methanol, are used as the biomass.

4. Process according to claim 1, characterised in that biomasses from the production of penicillin, tetracyclin or sisomycin are used.

5. Process according to claims 1 to 4, characterised in that component B) comprises a substantially monomer-free, cross-linked distillation residue having an NCO content of from 1 to 15%, by weight, and a particle size of less than 2 mm which is insoluble in inert organic solvents and cannot be melted without decomposing and which is obtained by (a) removing the monomeric tolylene diisocyanate from the crude phosgenation product of tolylene diamines, (b) optionally stirring the residue into water, (c) grinding and, optionally, (d) simultaneous and/or subsequent modification reactions with compounds reactive to the functional groups of the residue, and in that the denaturing reaction is carried out at temperatures between 70 and 200°C.

6. Process according to claims 1 to 5, characterised in that distillation residues B) are used which are brought to a particle size of less than 0.8 mm by stirring the residue into water, followed by grinding.

7. Process according to claims 1 to 6, characterised in that the distillation residues B) are used in amounts of 80 to 10% by weight, based on A) + B).

8. Process according to claims 1 to 7, characterised in that the denaturing reaction is carried out essentially in the absence of liquid components in a fluidised bed.

9. Process according to claims 1 to 8, characterised in that a condensation reaction of the biomass with carbonyl compounds and, optionally, other compounds capable of aminoplast and/or phenoplast formation is carried out before, after or simultaneously with the isocyanate polyaddition reaction.

10. Process according to claim 9, characteried in that the formaldehyde is used as the carbonyl compound.

11. Process according to claim 1 to 10, characterised in that organic and/or inorganic fillers are used.

12. Use of the denatured polyaddition products produced in accordance with claims 1 to 11 as plant nutrients.

13. Use of the denatured polyaddition products produced in accordance with claims 1 to 11 as heat-formable moulding compositions and as a reactive filler in the production of plastics by polyaddition, polycondensation and/or polymerisation reactions.

14. Use of the denatured polyaddition products produced in accordance with claims 1 to 11, optionally together with conventional formaldehyde resins, as binders, in amounts of 2 to 90% by weight, based on the total weight of the moulding, for the production of boards or mouldings by the hot-pressing of lignocellulose-containing starting materials.

**Revendications**

1. Procédé de production de produits dénaturés de polyaddition à partir de biomasses et d'isocyanates, avec addition éventuelle d'autres substances formant des polymères et traitement thermique subséquent, caractérisé en ce qu'on fait réagir

A) 5 à 98% en poids, par rapport à A) + B), d'une biomasse à base de micro-organismes ou de leurs dérivés et produits de décomposition avec

B) 95 à 2% en poids, par rapport à A) + B), de résidus de distillation obtenus dans la production industrielle d'isocyanates, éventuellement en présence

C) d'eau et/ou d'un solvant organique ainsi que, le cas échéant, en présence

D) d'additifs organiques et/ou inorganiques,

à des températures au-dessus de 50°C, avec dénaturation totale du composant A).

2. Procédé suivant la revendication 1, caractérisé en ce qu'on utilise comme biomasse des boues d'épuration aqueuses ou séchées, éventuellement déjà soumises à la digestion aérobie ou anaérobie, d'installations industrielles ou communales de clarification.

3. Procédé suivant la revendication 1, caractérisé en ce qu'on utilise comme biomasse des levures provenant de processus de fermentation industrielle pour la production d'alcool, d'acide acétique, d'acide lactique, d'acide citrique ou d'acide tartrique droit ou pour la production des protéines à partir de pétrole, de paraffine, de méthane ou de méthanol.

4. Procédé suivant la revendication 1, caractérisé en ce qu'on utilise des biomasses de la production de la pénicilline, de la tétracycline ou de la sisomycine.

5. Procédé suivant les revendications 1 à 4, caractérisé en ce qu'on utilise comme composant B) un résidu de distillation essentiellement dépourvu de monomères, réticulé, insoluble dans des solvants organiques inertes, fusible sans décomposition, ayant une teneur en NCO de 1 à 15% en poids et une grosseur de particules de moins de 2 mm, qui est obtenu par (a) élimination du diisocyanatotoluène monomérique du produit brut de phosgénation de diaminotoluènes, (b) éventuellement délayage du résidu dans l'eau, (c) broyage et, le cas échéant, (d) réactions simultanées et/ou subséquentes de modification avec des composés réactifs vis-à-vis des groupes fonctionnels du résidu, et en ce que la réaction de dénaturation est conduite à des températures comprises entre 70 et 200°C.

6. Procédé suivant les revendications 1 à 5, caractérisé en ce qu'on utilise des résidus de distillation B) qui sont réduits à une grosseur de particules de moins de 0,8 mm par délayage du résidu dans l'eau et broyage subséquent.

7. Procédé suivant les revendications 1 à 6, caractérisé en ce que les résidus de distillation B) sont utilisés en quantités de 80 à 10% en poids, par rapport à la somme A) + B).

8. Procédé suivant les revendications 1 à 7, caractérisé en ce que la réaction de dénaturation est conduite principalement en l'absence de composants liquides, en lit tourbillonnaire ou en lit fluide.

9. Procédé suivant les revendications 1 à 8, caractérisé en ce qu'une réaction de condensation de la biomasse avec des composés carbonyliques et, le cas échéant, avec d'autres composés aptes à la formation d'aminoplastes et/ou de phénoplastes, a lieu avant, après ou pendant la réaction de polyaddition d'un isocyanate.

10. Procédé suivant la revendication 9, caractérisé en ce qu'on utilise le formaldéhyde comme composé carbonylique.

11. Procédé suivant les revendications 1 à 10, caractérisé en ce qu'on utilise des charges organiques et/ou inorganiques.

12. Utilisation des produits dénaturés de polyaddition préparés selon les revendications 1 à 11 comme substance pour la nutrition des plantes.

13. Utilisation des produits dénaturés de polyaddition obtenus selon les revendications 1 à 11 comme matières à mouler déformables à la chaleur et comme charge réactive dans la production de matières plastiques par des réactions de polyaddition, de polycondensation et/ou de polymérisation.

14. Utilisation des produits dénaturés de polyaddition obtenus selon les revendications 1 à 11, le cas échéant conjointement avec des résines formaldéhyde classiques comme liant en quantités de 2 à 90 en poids par rapport au poids total de la pièce moulée, dans la production de plaques ou pièces moulées par compression à chaud de matières premières contenant de la lignocellulose.